(19) Europäisches Patentamt / European Patent Office / Office européen des brevets

(11) EP 4 317 134 A1

(12) **EUROPEAN PATENT APPLICATION**
published in accordance with Art. 153(4) EPC

(43) Date of publication:
**07.02.2024 Bulletin 2024/06**

(21) Application number: **22780657.7**

(22) Date of filing: **25.03.2022**

(51) International Patent Classification (IPC):
***C07D 209/86*** *(2006.01)* ***H01L 51/46*** *(2006.01)*

(52) Cooperative Patent Classification (CPC):
**H10K 85/50; C07D 209/86**

(86) International application number:
**PCT/JP2022/014732**

(87) International publication number:
**WO 2022/210445 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.03.2021 JP 2021057864**

(71) Applicant: **Hodogaya Chemical Co., Ltd.**
**Tokyo, 105-0021 (JP)**

(72) Inventors:
- **SAKURAI Atsushi**
  **Tokyo 105-0021 (JP)**
- **HASHIMOTO Maki**
  **Tokyo 105-0021 (JP)**
- **OHKURA Yuya**
  **Tokyo 105-0021 (JP)**
- **TAKAHASHI Hideaki**
  **Tokyo 105-0021 (JP)**
- **SATO Hiroshi**
  **Tokyo 105-0021 (JP)**
- **ITO Toshiaki**
  **Tokyo 105-0021 (JP)**

(74) Representative: **Müller-Boré & Partner Patentanwälte PartG mbB**
**Friedenheimer Brücke 21**
**80639 München (DE)**

(54) **COMPOUND HAVING SULFONATE GROUP, AND PHOTOELECTRIC CONVERSION ELEMENT USING SAID COMPOUND**

(57) Provided are a compound represented by the following Formula (1) useful as a hole transport material for a photoelectric conversion element, and a photoelectric conversion element using the compound in a hole transport layer.

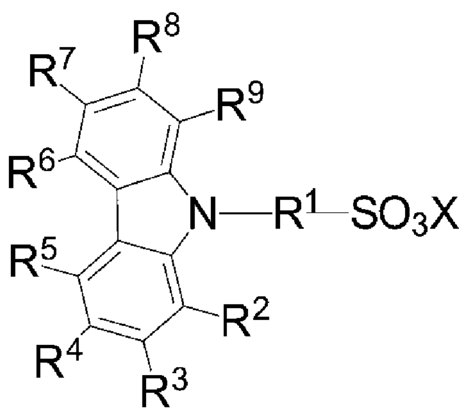

(1)

EP 4 317 134 A1

# FIGURE

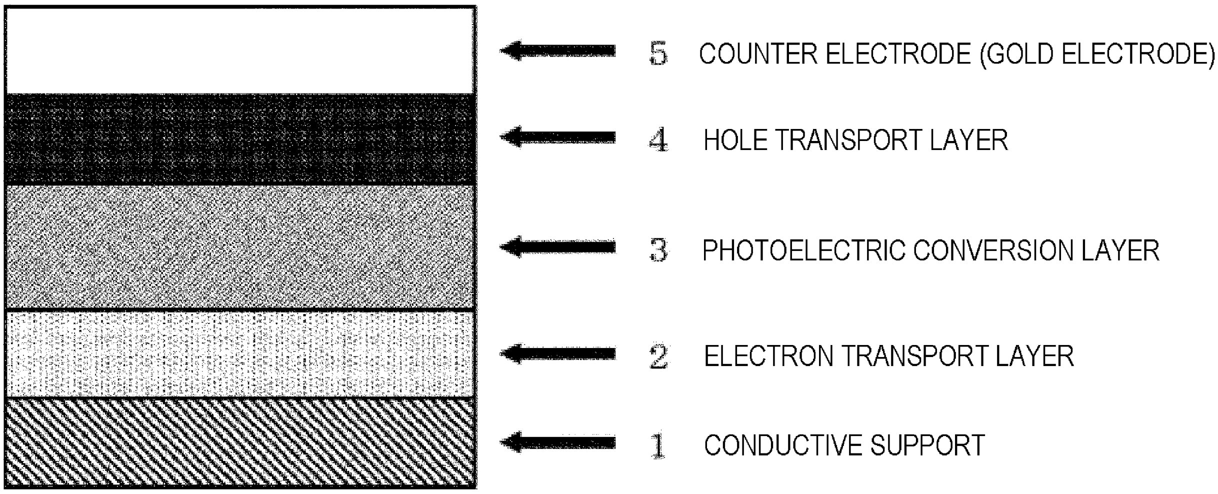
5 COUNTER ELECTRODE (GOLD ELECTRODE)
4 HOLE TRANSPORT LAYER
3 PHOTOELECTRIC CONVERSION LAYER
2 ELECTRON TRANSPORT LAYER
1 CONDUCTIVE SUPPORT

## Description

### TECHNICAL FIELD

The present invention relates to a compound having a sulfonate group, a photoelectric conversion element using the same, and a perovskite solar cell.

### BACKGROUND ART

In recent years, photovoltaics has attracted attention as clean energy, and solar cells have been actively developed. The development of a solar cell using a perovskite material as a photoelectric conversion layer (hereinafter, referred to as the perovskite solar cell) is attracting attention as a next-generation solar cell that can be manufactured at low cost by a solution process (for example, Patent Literature 1, and Non Patent Literatures 1 to 2).

The perovskite solar cell often uses a hole transport material in an element. The objects to use the hole transport material include (1) improving a photoelectric conversion efficiency and (2) protecting a perovskite material that is susceptible to moisture and oxygen (for example, Non Patent Literature 3). Spiro-OMeTAD is often used as a standard hole transport material, and there are few reports of the hole transport material that contributes to photoelectric conversion characteristics more than Spiro-OMeTAD.

When a hole transport material is used in an element of the perovskite solar cell, in a method in the related art for producing an element using an organic compound as the hole transport material, a dopant, which is an additive, is added to the hole transport material. The dopant is used to reduce an electrical resistance of the hole transport material (for example, Non Patent Literatures 3 to 4).

However, the use of the dopant, which is the additive, not only complicates a manufacturing process, but also increases manufacturing costs. Acceleration of moisture absorption and corrosion of a photoelectric conversion layer due to dopants, and deterioration of a hole transport layer due to volatilization of dopants are reported, and these lead to deterioration of element characteristics (for example, Non Patent Literature 5). Therefore, it is desired to develop a photoelectric conversion element having a hole transport layer containing no dopant and exhibiting high photoelectric conversion characteristics and durability. On the other hand, in some cases, a dopant is unavoidably used in order to obtain a good photoelectric conversion efficiency. Therefore, it is desired to develop a photoelectric conversion element that exhibits little deterioration in element characteristics over time even if a dopant is contained.

### CITATION LIST

#### PATENT LITERATURE

Patent Literature 1: WO 2017/104792

#### NON PATENT LITERATURE

Non Patent Literature 1: J. Am. Chem. Soc., 2009, Volume 131, P. 6050 to 6051
Non Patent Literature 2: Science, 2012, Volume 388, P. 643 to 647
Non Patent Literature 3: Chem. Sci., 2019, 10, P. 6748 to 6769
Non Patent Literature 4: Adv. Funct. Mater, 29, 24, 2019, 1901296
Non Patent Literature 5: J. Am. Chem. Soc., 2018, 140, 48, 16720 to 16730

### SUMMARY OF INVENTION

#### TECHNICAL PROBLEM

The problem to be solved by the present invention is to provide a compound that is useful as a hole transport material for a photoelectric conversion element containing no dopant, which is an additive, and a photoelectric conversion element and a perovskite solar cell that use the compound for a hole transport layer and has good photoelectric conversion characteristics. The problem to be solved by the present invention is also to provide a compound useful as a hole transport material for a photoelectric conversion element that exhibits little deterioration in element characteristics over time even if a dopant is contained.

SOLUTION TO PROBLEM

In order to solve the above problem, as a result of intensive studies on improving photoelectric conversion characteristics, the inventors have found that the use of a compound having a specific structure as a hole transport layer provides a highly efficient photoelectric conversion element and a perovskite solar cell. That is, the gist of the present invention is as follows.

1. A compound represented by the following Formula (1).

[Chem. 1]

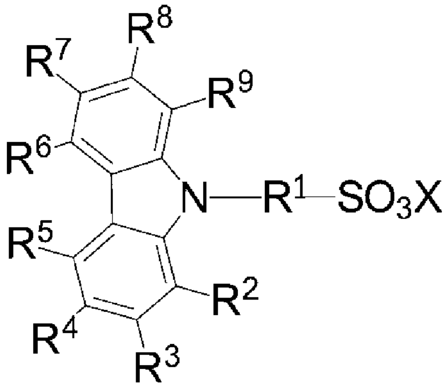

(1)

[In the formula, $R^1$ represents:

a linear or branched alkyl group having 1 to 18 carbon atoms that optionally has a substituent group;
a linear or branched alkenyl group having 2 to 20 carbon atoms that optionally has a substituent group;
a linear or branched alkynyl group having 2 to 20 carbon atoms that optionally has a substituent group;
a cycloalkyl group having 3 to 12 carbon atoms that optionally has a substituent group;
an aromatic hydrocarbon group having 6 to 36 carbon atoms that optionally has a substituent group; or a heterocyclic group having 5 to 36 ring atoms that optionally has a substituent group,
X represents a monovalent cation excluding a hydrogen ion, and
$R^2$ to $R^9$ each independently represent:

a hydrogen atom;
a linear or branched alkyl group having 1 to 18 carbon atoms that optionally has a substituent group;
a linear or branched alkenyl group having 2 to 20 carbon atoms that optionally has a substituent group;
a linear or branched alkynyl group having 2 to 20 carbon atoms that optionally has a substituent group;
a cycloalkyl group having 3 to 12 carbon atoms that optionally has a substituent group;
a linear or branched alkoxy group having 1 to 20 carbon atoms that optionally has a substituent group;
a cycloalkoxy group having 3 to 10 carbon atoms that optionally has a substituent group;
a linear or branched alkoxycarbonyl group having 1 to 18 carbon atoms that optionally has a substituent group;
a thio group having 1 to 18 carbon atoms that optionally has a substituent group;
an amino group having 1 to 20 carbons that optionally has a substituent group;
an aromatic hydrocarbon group having 6 to 36 carbon atoms that optionally has a substituent group; or
a heterocyclic group having 5 to 36 ring atoms that optionally has a substituent group].

2. The compound according to 1 above, in which in Formula (1), $R^1$ represents the linear or branched alkyl group having 1 to 18 carbon atoms that optionally has a substituent group.
3. The compound according to 1 or 2 above, in which in Formula (1), at least one of $R^2$ to $R^9$ represents the aromatic hydrocarbon group having 6 to 36 carbon atoms that optionally has a substituent group or the amino group having 1 to 20 carbons that optionally has a substituent group.
4. The compound according to any one of 1 to 3 above, in which in Formula (1), X represents an alkali metal ion or an ammonium ion that optionally has a substituent group.
5. The compound according to 4 above, in which in Formula (1), the alkali metal ion is at least one selected from the group consisting of a sodium ion, a potassium ion, a rubidium ion, and a cesium ion.

6. A hole transport material containing the compound according to any one of 1 to 5 above.
7. A hole transport material composition for a photoelectric conversion element containing the hole transport material according to 6 above.
8. A photoelectric conversion element using the hole transport material composition for photoelectric conversion according to 7 above.

ADVANTAGEOUS EFFECTS OF INVENTION

A compound having a sulfonate group and a hole transport layer using the same according to the present invention allows for providing a perovskite solar cell and a photoelectric conversion element having a good photoelectric conversion efficiency by using the compound between the photoelectric conversion layer and an electrode.

BRIEF DESCRIPTION OF DRAWINGS

The FIGURE is a schematic cross-sectional view showing a configuration of a photoelectric conversion element according to an example and a comparative example of the present invention.

DESCRIPTION OF EMBODIMENTS

Hereinafter, embodiments of the present invention will be described in detail. A hole transport layer of the present invention is used in a photoelectric conversion element and a perovskite photoelectric conversion element.
The compound represented by Formula (1) is specifically described below, but the present invention is not limited thereto.
In Formula (1), $R^1$ represents:

a linear or branched alkyl group having 1 to 18 carbon atoms that optionally has a substituent group;
a linear or branched alkenyl group having 2 to 20 carbon atoms that optionally has a substituent group;
a linear or branched alkynyl group having 2 to 20 carbon atoms that optionally has a substituent group;
a cycloalkyl group having 3 to 12 carbon atoms that optionally has a substituent group;
an aromatic hydrocarbon group having 6 to 36 carbon atoms that optionally has a substituent group; or
a heterocyclic group having 5 to 36 ring atoms that optionally has a substituent group.

In Formula (1), specific examples of the "linear or branched alkyl group having 1 to 18 carbon atoms" in the "linear or branched alkyl group having 1 to 18 carbon atoms that optionally has a substituent group" represented by $R^1$ may include a methyl group, an ethyl group, an n-propyl group, an isopropyl group, an n-butyl group, an isobutyl group, an s-butyl group, a t-butyl group, an n-pentyl group, an isopentyl group, an n-hexyl group, a 2-ethylhexyl group, a heptyl group, an octyl group, an isooctyl group, a nonyl group, and a decyl group. In $R^1$, one of hydrogens in the groups listed above is substituted with a sulfonate group ($-SO_3X$).
In Formula (1), specific examples of the "linear or branched alkenyl group having 2 to 20 carbon atoms" in the "linear or branched alkenyl group having 2 to 20 carbon atoms that optionally has a substituent group" represented by $R^1$ may include a vinyl group, a 1-propenyl group, an allyl group, a 1-butenyl group, a 2-butenyl group, a 1-pentenyl group, a 1-hexenyl group, an isopropenyl group, an isobutenyl group, or a linear or branched alkenyl group having 2 to 20 carbon atoms in which a plurality of these alkenyl groups are bonded. In $R^1$, one of hydrogens in the groups listed above is substituted with a sulfonate group ($-SO_3X$).
In Formula (1), specific examples of the "linear or branched alkynyl group having 2 to 20 carbon atoms" in the "linear or branched alkynyl group having 2 to 20 carbon atoms that optionally has a substituent group" represented by $R^1$ may include an ethynyl group, a 1-propynyl group, a 2-propynyl group, a 1-butynyl group, a 2-butynyl group, a 1-methyl-2-propynyl group, a 1-pentynyl group, a 2-pentynyl group, a 1-methyl-n-butynyl group, a 2-methyl-n-butynyl group, a 3-methyl-n-butynyl group, and a 1 -hexynyl group. In $R^1$, one of hydrogens in the groups listed above is substituted with a sulfonate group ($-SO_3X$).
In Formula (1), specific examples of the "cycloalkyl group having 3 to 12 carbon atoms" in the "cycloalkyl group having 3 to 12 carbon atoms that optionally has a substituent group" represented by $R^1$ may include a cyclopropyl group, a cyclobutyl group, a cyclopentyl group, a cyclohexyl group, a cycloheptyl group, a cyclooctyl group, a cyclodecyl group, and a cyclododecyl group. In $R^1$, one of hydrogens in the groups listed above is substituted with a sulfonate group ($-SO_3X$).
In Formula (1), specific examples of the "aromatic hydrocarbon group having 6 to 36 carbon atoms" in the "aromatic hydrocarbon group having 6 to 36 carbon atoms that optionally has a substituent group" represented by $R^1$ may include a phenyl group, a biphenyl group, a terphenyl group, a naphthyl group, a biphenyl group, an anthracenyl group (anthryl group), a phenanthryl group, a fluorenyl group, an indenyl group, a pyrenyl group, a perylenyl group, a

fluoranthenyl group, and a triphenylenyl group. In $R^1$, one of hydrogens in the groups listed above is substituted with a sulfonate group ($-SO_3X$). In the present invention, the aromatic hydrocarbon group includes a "condensed polycyclic aromatic group"

In Formula (1), specific examples of the "heterocyclic group having 5 to 36 ring atoms" in the "heterocyclic group having 5 to 36 ring atoms that optionally has a substituent group" represented by $R^1$ may include a pyridyl group, a pyrimidylinyl group, a triazinyl group, a thienyl group, a furyl group (furanyl group), a pyrrolyl group, an imidazolyl group, a pyrazolyl group, a triazolyl group, a quinolyl group, a isoquinolyl group, a naphthyldinyl group, an acridinyl group, a phenanthrolinyl group, a benzofuranyl group, a benzothienyl group, an oxazolyl group, an indolyl group, a carbazolyl group, a benzoxazolyl group, a thiazolyl group, benzothiazolyl group, a quinoxalinyl group, a benzimidazolyl group, a pyrazolyl group, a dibenzofuranyl group, a dibenzothienyl group, and a carbonylyl group. In $R^1$, one of hydrogens in the groups listed above is substituted with a sulfonate group ($-SO_3X$).

In Formula (1), specific examples of the " substituent group" in the "linear or branched alkyl group having 1 to 18 carbon atoms that optionally has a substituent group", the "linear or branched alkenyl group having 2 to 20 carbon atoms that optionally has a substituent group", the "linear or branched alkynyl group having 2 to 20 carbon atoms that optionally has a substituent group" the "cycloalkyl group having 3 to 12 carbon atoms that optionally has a substituent group", the "aromatic hydrocarbon group having 6 to 36 carbon atoms that optionally has a substituent group", or the "heterocyclic group having 5 to 36 ring atoms that optionally has a substituent group" represented by $R^1$ may include:

a halogen atom such as a fluorine atom, a chlorine atom, a bromine atom, and an iodine atom; a cyano group; a hydroxyl group; a nitro group; a nitroso group; a carboxyl group; a phosphate group;
a carboxylic acid ester group such as a methyl ester group and an ethyl ester group;
a linear or branched alkyl group having 1 to 18 carbon atoms such as a methyl group, an ethyl group, an n-propyl group, an isopropyl group, an n-butyl group, an isobutyl group, an s-butyl group, a t-butyl group, an n-pentyl group, an isopentyl group, an n-hexyl group, a 2-ethylhexyl group, a heptyl group, an octyl group, an isooctyl group, a nonyl group, and a decyl group;
a linear or branched alkenyl group having 2 to 20 carbon atoms such as a vinyl group, a 1-propenyl group, an allyl group, a 1-butenyl group, a 2-butenyl group, a 1-pentenyl group, a 1-hexenyl group, an isopropenyl group, and an isobutenyl group;
a linear or branched alkoxy group having 1 to 18 carbon atoms such as a methoxy group, an ethoxy group, a propoxy group, a t-butoxy group, a pentyloxy group, and a hexyloxy group;
an aromatic hydrocarbon group having 6 to 30 carbon atoms such as a phenyl group, a naphthyl group, an anthryl group, a phenanthryl group, and a pyrenyl group;
a heterocyclic group having 5 to 30 ring atoms such as a pyridyl group, a pyrimidylinyl group, a triazinyl group, a thienyl group, a furyl group (furanyl group), a pyrrolyl group, an imidazolyl group, a pyrazolyl group, a triazolyl group, a quinolyl group, a isoquinolyl group, a naphthyldinyl group, an acridinyl group, a phenanthrolinyl group, a benzofuranyl group, a benzothienyl group, an oxazolyl group, an indolyl group, a carbazolyl group, a benzoxazolyl group, a thiazolyl group, benzothiazolyl group, a quinoxalinyl group, a benzimidazolyl group, a pyrazolyl group, a dibenzofuranyl group, a dibenzothienyl group, and a carbonylyl group;
an amino group having 0 to 18 carbon atoms such as an unsubstituted amino group ($-NH_2$), a monosubstituted amino group such as an ethylamino group, an acetylamino group, and a phenylamino group, and a disubstituted amino group such as a diethylamino group, a diphenylamino group, and an acetylphenylamino group; and
a thio group having 0 to 18 carbon atoms such as an unsubstituted thio group (thiol group: -SH), a methylthio group, an ethylthio group, a propylthio group, a hex-5-ene-3-thio group, a phenylthio group, and a biphenylthio group. Only one of the "substituent groups" may be included, or two or more "substituent groups" may be included in the above groups. When the two or more "substituent groups" are included, the substituent groups may be the same or different. The "substituent groups" may further have the substituent groups exemplified above.

In Formula (1), X in the sulfonate group ($-SO_3X$) of $R^1$ represents a monovalent cation excluding a hydrogen ion. Specifically, the monovalent cation is preferably an alkali metal ion, an ammonium ion that optionally has a substituent group, or a phosphonium ion that optionally has a substituent group, more preferably an alkali metal ion or an ammonium ion that optionally has a substituent group, and particularly preferably an alkali metal ion, but the present invention is not limited thereto.

Specific examples of an alkali metal include a lithium ion, a sodium ion, a potassium ion, a rubidium ion, a cesium ion, and a francium ion, and the alkali metal is preferably at least one selected from the group consisting of the sodium ion, the potassium ion, the rubidium ion, and the cesium ion.

Specific examples of the ammonium ion that optionally has a substituent group include a methylammonium ion, a methylammonium monofluoride ion, a methylammonium difluoride ion, a methylammonium trifluoride ion, an ethylammonium ion, an isopropylammonium ion, an n-propylammonium ion, an isobutylammonium ion, an n-butylammonium ion, a t-butylammonium ion, a dimethylammonium ion, a diethylammonium ion, a phenylammonium ion, a benzylammonium ion, a phenethylammonium ion, a guanidinium ion, a formamidinium ion, an acetamidinium ion, an imidazolium ion, a tri-n-butylammonium ion, and a tetra-n-butylammonium ion.

in Formula (1), $R^1$ preferably represents the linear or branched alkyl group having 1 to 18 carbon atoms that optionally has a substituent group. $R^1$ more preferably represents an alkyl group having 18 or less carbon atoms in total that optionally includes a substituent group, still more preferably represents an alkyl group having 10 or less carbon atoms in total that optionally includes a substituent group, and particularly preferably represents an alkyl group having 6 or less carbon atoms in total that optionally includes a substituent group.

In Formula (1), $R^2$ to $R^9$ each independently represent:

a hydrogen atom;
a linear or branched alkyl group having 1 to 18 carbon atoms that optionally has a substituent group;
a linear or branched alkenyl group having 2 to 20 carbon atoms that optionally has a substituent group;
a linear or branched alkynyl group having 2 to 20 carbon atoms that optionally has a substituent group;
a cycloalkyl group having 3 to 12 carbon atoms that optionally has a substituent group;
a linear or branched alkoxy group having 1 to 20 carbon atoms that optionally has a substituent group;
a cycloalkoxy group having 3 to 10 carbon atoms that optionally has a substituent group;
a linear or branched alkoxycarbonyl group having 1 to 18 carbon atoms that optionally has a substituent group;
a thio group having 1 to 18 carbon atoms that optionally has a substituent group;
an amino group having 1 to 20 carbons that optionally has a substituent group;
an aromatic hydrocarbon group having 6 to 36 carbon atoms that optionally has a substituent group; or
a heterocyclic group having 5 to 36 ring atoms that optionally has a substituent group.

In Formula (1), examples of the "linear or branched alkyl group having 1 to 18 carbon atoms" in the "linear or branched alkyl group having 1 to 18 carbon atoms that optionally has a substituent group" represented by $R^2$ to $R^9$ may include the same groups as those of the "linear or branched alkyl group having 1 to 18 carbon atoms that optionally has a substituent group" represented by $R^1$ in Formula (1).

In Formula (1), examples of the "linear or branched alkenyl group having 2 to 20 carbon atoms" in the "linear or branched alkenyl group having 2 to 20 carbon atoms that optionally has a substituent group" represented by $R^2$ to $R^9$ may include the same groups as those of the "linear or branched alkenyl group having 2 to 20 carbon atoms" represented by $R^1$ in Formula (1).

In Formula (1), examples of the "linear or branched alkynyl group having 2 to 20 carbon atoms" in the "linear or branched alkynyl group having 2 to 20 carbon atoms that optionally has a substituent group" represented by $R^2$ to $R^9$ may include the same groups as those of the "linear or branched alkynyl group having 2 to 20 carbon atoms" represented by $R^1$ in Formula (1).

In Formula (1), examples of the "cycloalkyl group having 3 to 12 carbon atoms" in the "cycloalkyl group having 3 to 12 carbon atoms that optionally has a substituent group" represented by $R^2$ to $R^9$ may include the same groups as those of the "cycloalkyl group having 3 to 12 carbon atoms" represented by $R^1$ in Formula (1).

In Formula (1), specific examples of the "linear or branched alkoxy group having 1 to 20 carbon atoms" in the "linear or branched alkoxy group having 1 to 20 carbon atoms that optionally has a substituent group" represented by $R^2$ to $R^9$ may include a methoxy group, an ethoxy group, a propoxy group, an n-butoxy group, an n-pentyloxy group, n-hexyloxy group, a heptyloxy group, an octyloxy group, a nonyloxy group, a decyloxy group, an isopropoxy group, an isobutoxy group, an s-butoxy group, t-butoxy group, an isooctyloxy group, a t-octyloxy group, a phenoxy group, a tolyloxy group, a biphenylyloxy group, a terphenylyloxy group, a naphthyloxy group, an anthryloxy group, a phenanthryloxy group, a fluorenyloxy group, and an indenyloxy group.

In Formula (1), specific examples of the "linear or branched cycloalkoxy group having 3 to 10 carbon atoms" in the "linear or branched cycloalkoxy group having 3 to 10 carbon atoms that optionally has a substituent group" represented by $R^2$ to $R^9$ may include a cyclopropoxy group, a cyclobutoxy group, a cyclopentyloxy group, and a cyclohexyloxy group.

In Formula (1), specific examples of the "linear or branched alkoxycarbonyl group having 1 to 18 carbon atoms" in the "linear or branched alkoxycarbonyl group having 1 to 18 carbon atoms that optionally has a substituent group" represented by $R^2$ to $R^9$ may include a methoxycarbonyl group and an ethoxycarbonyl group.

In Formula (1), specific examples of the "thio group having 1 to 18 carbon atoms" in the "thio group having 1 to 18 carbon atoms that optionally has a substituent group" represented by $R^2$ to $R^9$ may include a methylthio group, an ethylthio group, a propylthio group, a phenylthio group, and a biphenylthio group.

In Formula (1), specific examples of the "amino group having 1 to 20 carbon atoms" in the "amino group having 1 to 20 carbon atoms that optionally has a substituent group" represented by $R^2$ to $R^9$ may include a monosubstituted amino group such as an ethylamino group, an acetylamino group, and a phenylamino group, and a disubstituted amino

group such as a diethylamino group, a diphenylamino group, and an acetylphenylamino group.

In Formula (1), examples of the "aromatic hydrocarbon group having 6 to 36 carbon atoms" in the "aromatic hydrocarbon group having 6 to 36 carbon atoms that optionally has a substituent group" represented by $R^2$ to $R^9$ may include the same groups as those of the " aromatic hydrocarbon group having 6 to 36 carbon atoms that optionally has a substituent group" represented by $R^1$ in Formula (1).

In Formula (1), examples of the "heterocyclic group having 5 to 36 ring atoms" in the "heterocyclic group having 5 to 36 ring atoms that optionally has a substituent group" represented by $R^2$ to $R^9$ may include the same groups as those of the "heterocyclic group having 5 to 36 ring atoms that optionally has a substituent group" represented by $R^1$ in Formula (1).

In Formula (1), examples of the "substituent group" in the "linear or branched alkyl group having 1 to 18 carbon atoms that optionally has a substituent group", the "linear or branched alkenyl group having 2 to 20 carbon atoms that optionally has a substituent group", the "linear or branched alkynyl group having 2 to 20 carbon atoms that optionally has a substituent group", the "cycloalkyl group having 3 to 12 carbon atoms that optionally has a substituent group", the "linear or branched alkoxy group having 1 to 20 carbon atoms that optionally has a substituent group", the "cycloalkoxy group having 3 to 10 carbon atoms that optionally has a substituent group", the "linear or branched alkoxycarbonyl group having 1 to 18 carbon atoms that optionally has a substituent group", the "thio group having 1 to 18 carbon atoms that optionally has a substituent group", the "amino group having 1 to 20 carbons that optionally has a substituent group", the "aromatic hydrocarbon group having 6 to 36 carbon atoms that optionally has a substituent group", or the "heterocyclic group having 5 to 36 ring atoms that optionally has a substituent group" represented by $R^2$ to $R^9$ may include the same groups as those of the "linear or branched alkyl group having 1 to 18 carbon atoms that optionally has a substituent group" and the like represented by $R^1$ in Formula (1).

In Formula (1), at least one of $R^2$ to $R^9$ preferably represents the aromatic hydrocarbon group having 6 to 36 carbon atoms that optionally has a substituent group or the amino group having 1 to 20 carbons that optionally has a substituent group. In Formula (1), at least two of $R^2$ to $R^9$ more preferably represent the aromatic hydrocarbon group having 6 to 36 carbon atoms that optionally has a substituent group or the amino group having 1 to 20 carbons that optionally has a substituent group.

In Formula (1), each of $R^3$ and $R^8$ more preferably represents the aromatic hydrocarbon group having 6 to 36 carbon atoms that optionally has a substituent group or the amino group having 1 to 20 carbons that optionally has a substituent group, or each of $R^4$ and $R^7$ more preferably represents the aromatic hydrocarbon group having 6 to 36 carbon atoms that optionally has a substituent group or the amino group having 1 to 20 carbons that optionally has a substituent group. In this case, the remaining $R^2$, $R^4$ to $R^7$, and $R^9$, or the remaining $R^2$, $R^3$, $R^5$, $R^6$, $R^8$, and $R^9$ preferably represent a hydrogen atom.

In Formula (1), each of $R^3$ and $R^8$ more preferably represents a diphenylamino group that optionally has a substituent group or an aromatic hydrocarbon group having 6 to 36 carbon atoms substituted with a carbazole group or the diphenylamino group that optionally has a substituent group, or each of $R^4$ and $R^7$ more preferably represents a diphenylamino group that optionally has a substituent group or an aromatic hydrocarbon group having 6 to 36 carbon atoms substituted with a carbazole group or the diphenylamino group that optionally has a substituent group. In this case, the remaining $R^2$, $R^4$ to $R^7$, and $R^9$, or the remaining $R^2$, $R^3$, $R^5$, $R^6$, $R^8$, and $R^9$ preferably represent a hydrogen atom. In this case, $R^3$ and $R^8$ or $R^4$ and $R^7$ preferably have 50 or less, more preferably 40 or less, even more preferably 30 or less carbon atoms in total including a substituent group.

Specific examples of the compound represented by Formula (1) of the present invention are shown below, but the present invention is not limited thereto. In addition, the following exemplified compounds are described with some hydrogen atoms, carbon atoms, and the like omitted, and are examples of possible isomers, and include all other isomers. Each compound may be a mixture of two or more isomers.

[Chem. 2]

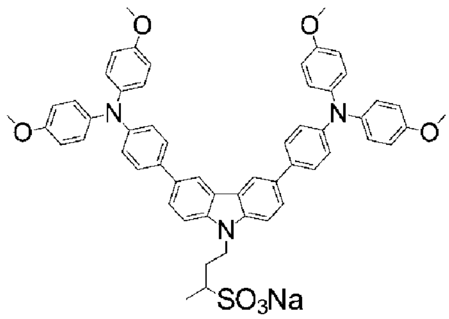

(A－1)

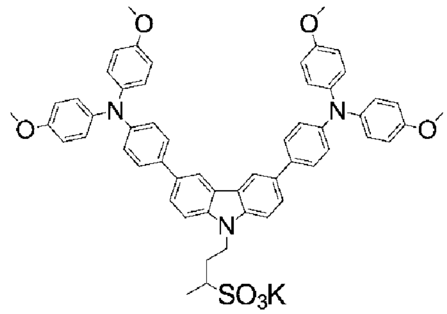

(A－2)

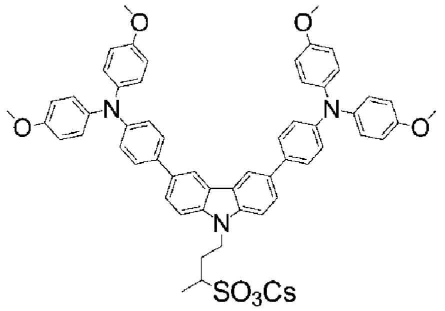

(A－3)

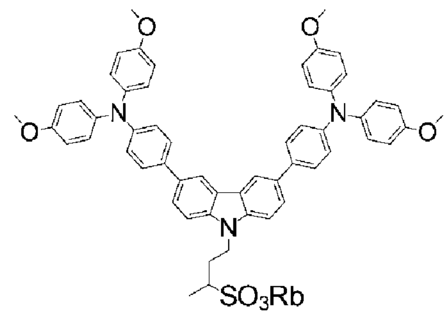

(A－4)

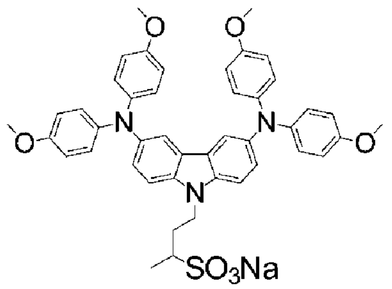

(A－5)

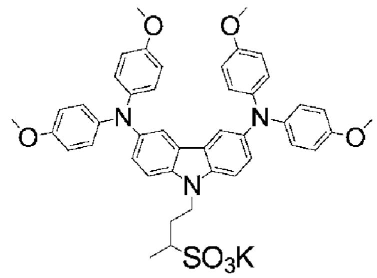

(A－6)

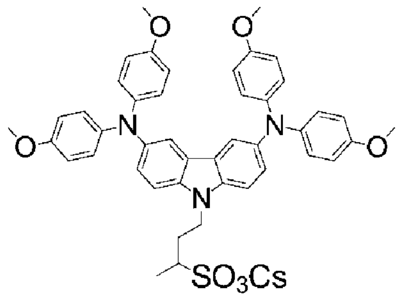

(A－7)

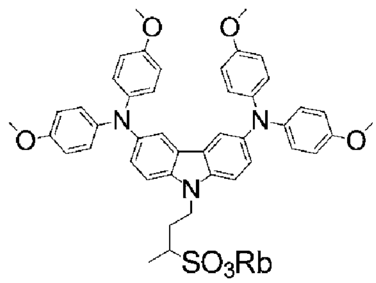

(A－8)

[Chem. 3]

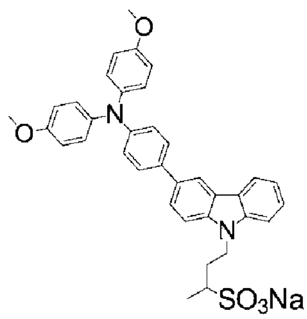

(A-9)

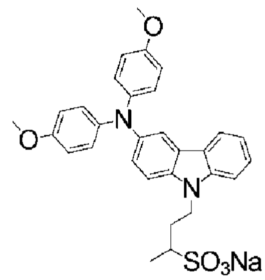

(A-10)

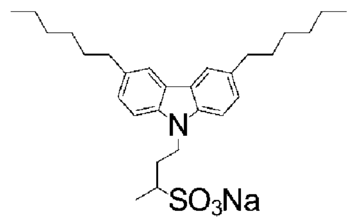

(A-11)

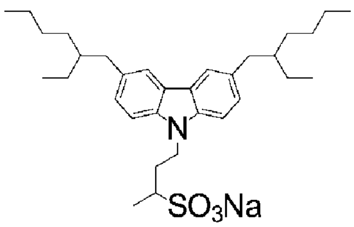

(A-12)

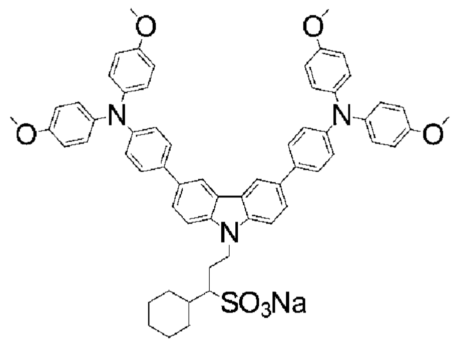

(A-13)

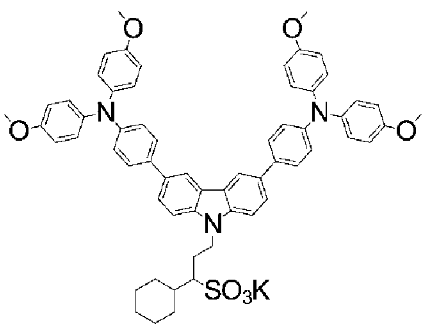

(A-14)

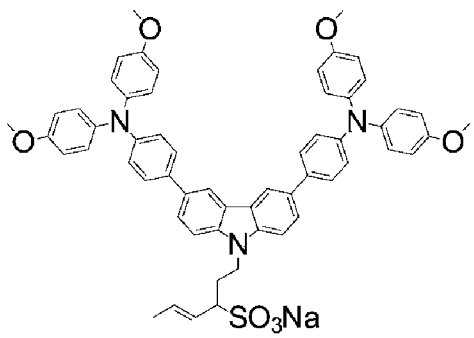

(A-15)

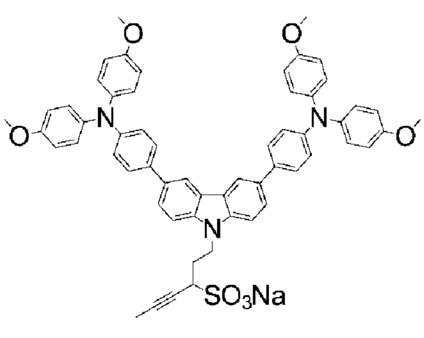

(A-16)

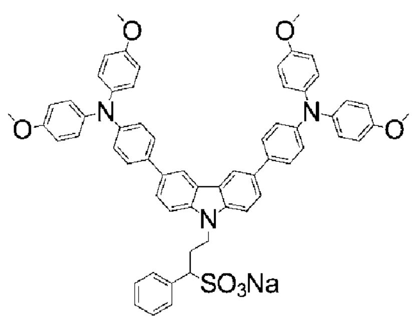

(A-17)

[Chem. 4]

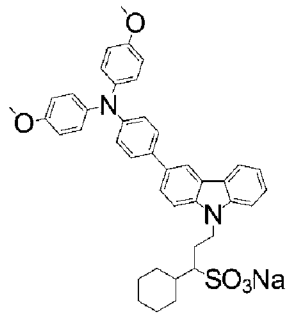

(A−18)

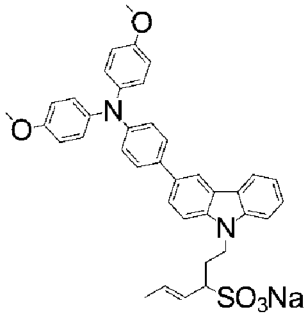

(A−19)

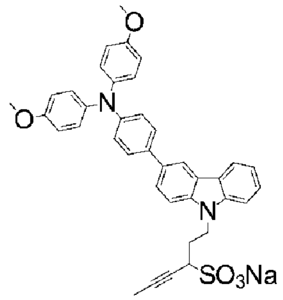

(A−20)

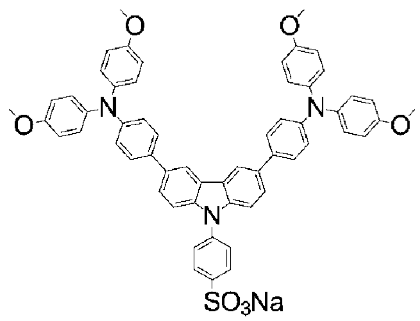

(A−21)

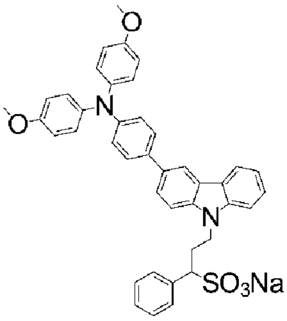

(A−22)

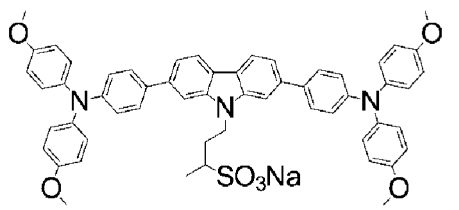

(A−23)

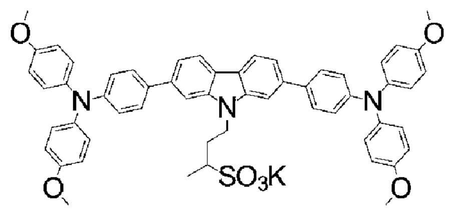

(A−24)

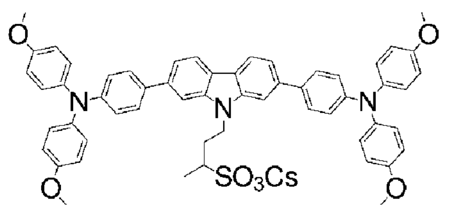

(A−25)

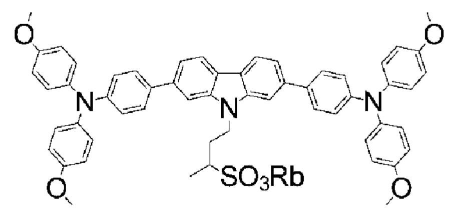

(A−26)

[Chem. 5]

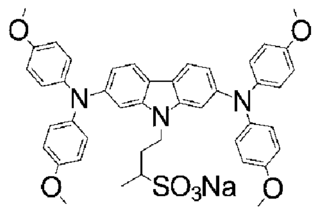

(A−27)

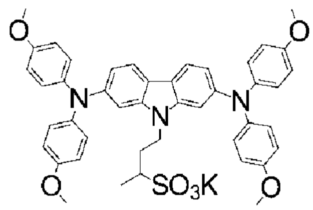

(A−28)

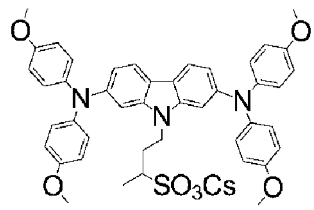

(A−29)

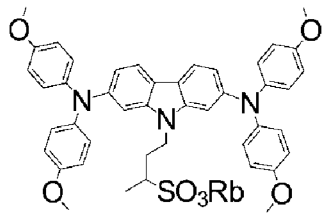

(A−30)

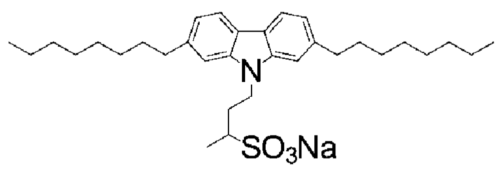

(A−31)

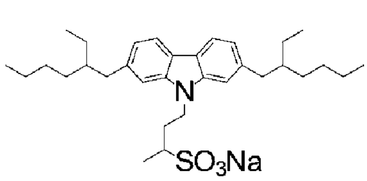

(A−32)

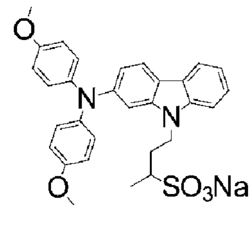

(A−33)

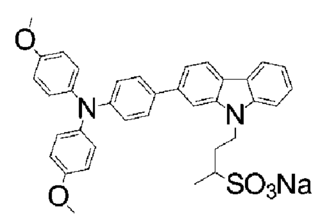

(A−34)

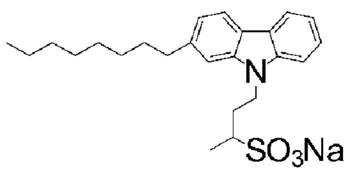

(A−35)

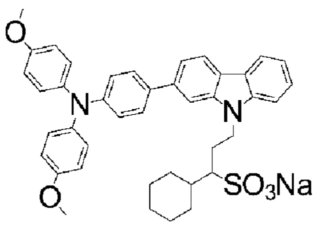

(A−36)

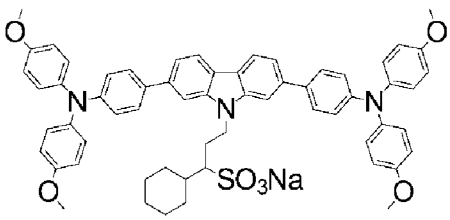

(A−37)

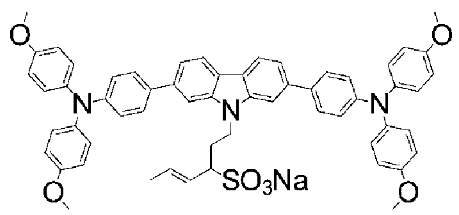

(A−38)

[Chem. 6]

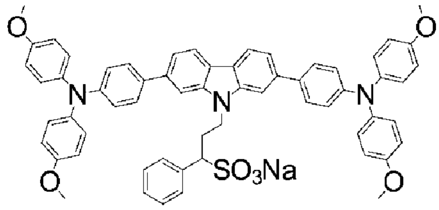

(A－39)

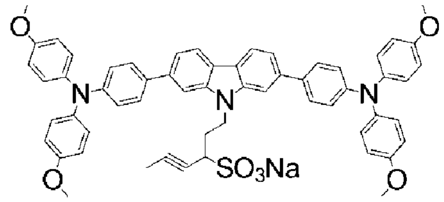

(A－40)

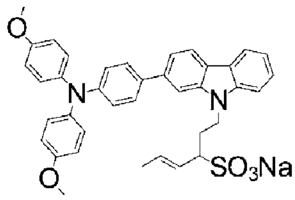

(A－41)

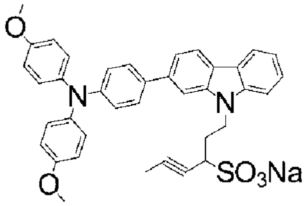

(A－42)

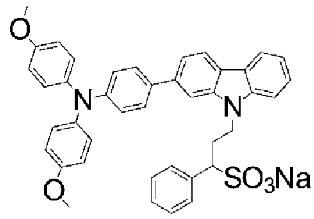

(A－43)

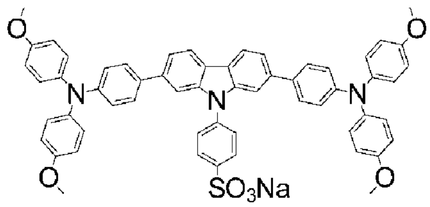

(A－44)

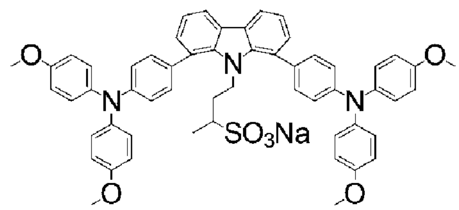

(A－45)

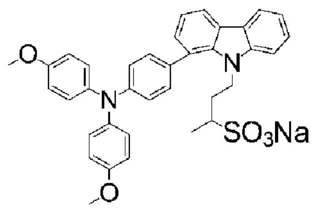

(A－46)

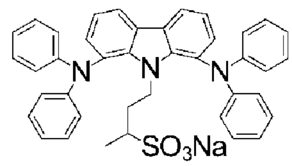

(A－47)

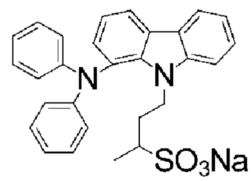

(A－48)

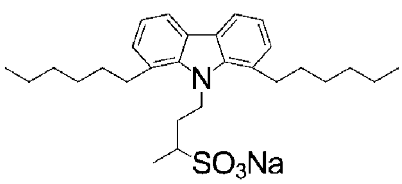

(A－49)

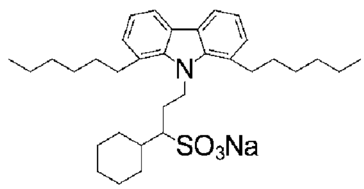

(A－50)

[Chem. 7]

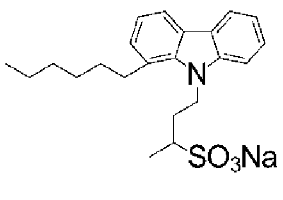

(A-51)

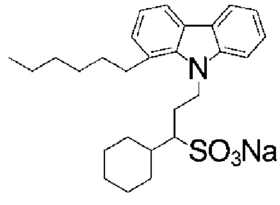

(A-52)

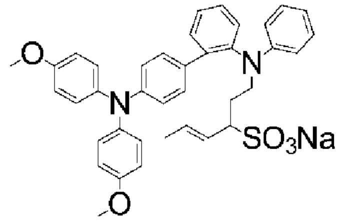

(A-53)

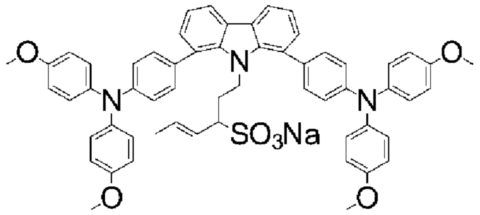

(A-54)

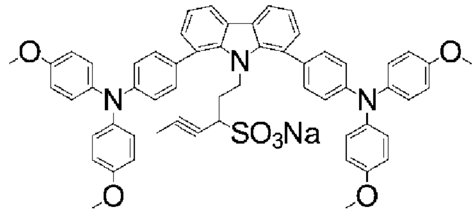

(A-55)

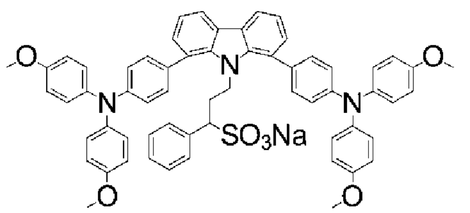

(A-56)

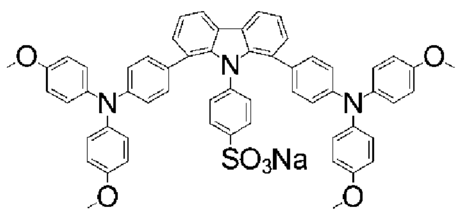

(A-57)

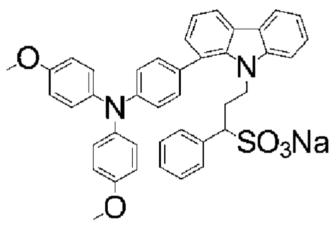

(A-58)

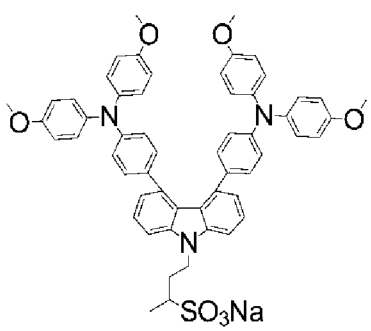

(A-59)

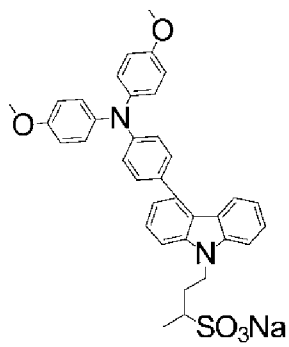

(A-60)

[Chem. 8]

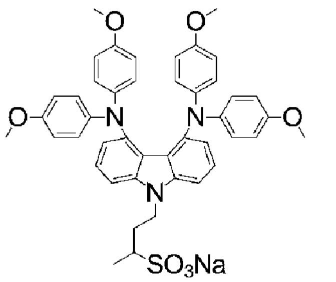
(A−61)

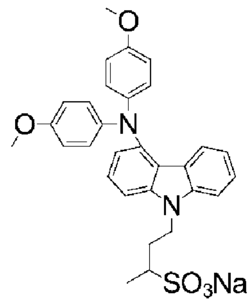
(A−62)

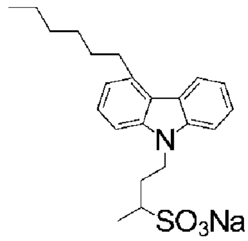
(A−63)

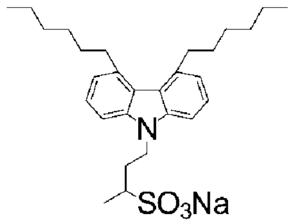
(A−64)

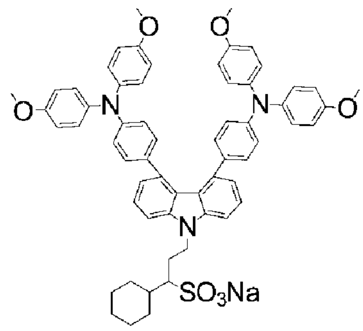
(A−65)

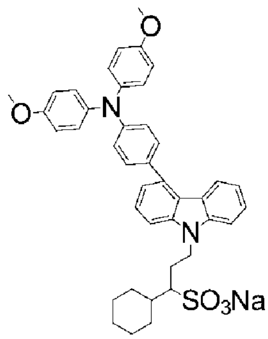
(A−66)

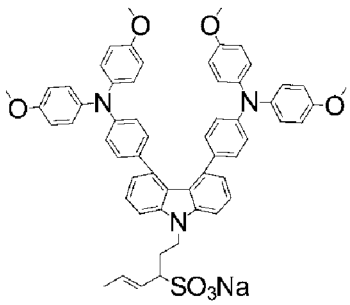
(A−67)

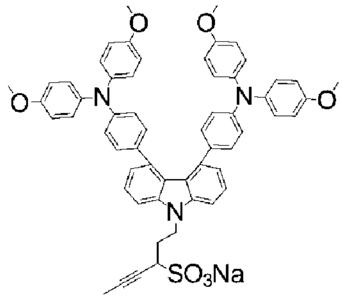
(A−68)

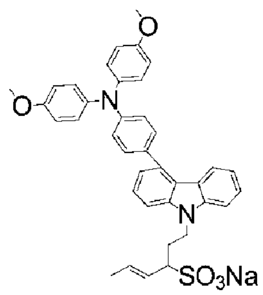
(A−69)

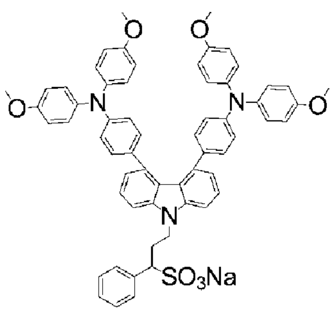
(A−70)

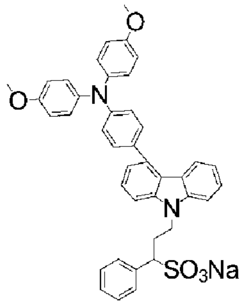
(A−71)

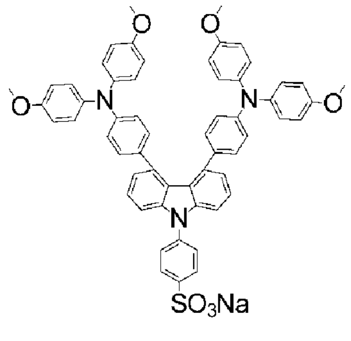
(A−72)

[Chem. 9]

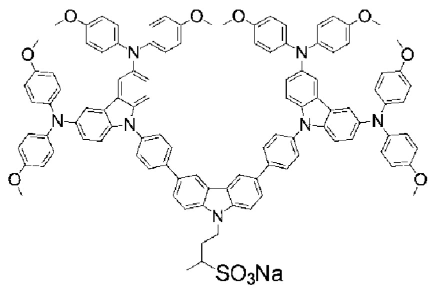

(A-73)

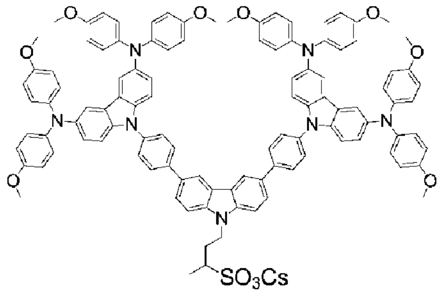

(A-74)

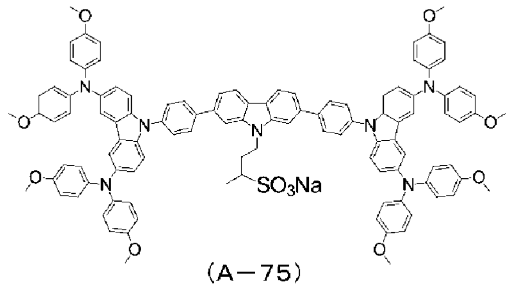

(A-75)

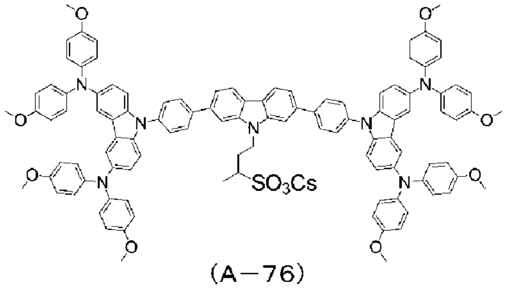

(A-76)

The compounds represented by Formula (1) of the present invention can be synthesized by a well-known method such as a method of Japanese Patent Application No. 2018-135255. A case of synthesizing the compound (A-1) will be described as an example. The compound (A-1) can be obtained by introducing a corresponding substituent group to 3,6-dibromocarbazole by a Suzuki-Miyaura coupling reaction or Buchwald reaction and then allowing the substituent group to react with a corresponding sultone. Also, a halogenated carbazole derivative is used as a precursor, and the compounds represented by Formula (1) can be obtained by a well-known method.

As a method for purifying the compounds represented by Formula (1) of the present invention, purification by column chromatography, purification by adsorption using silica gel, activated carbon, activated clay, or the like, recrystallization or crystallization with a solvent, or the like can be performed. Alternatively, it is effective to use these methods in combination to use a compound with increased purity. Identification of these compounds can be performed by nuclear magnetic resonance spectroscopy (NMR).

Each of the compounds represented by Formula (1) of the present invention can be used as a hole transport material contained in a hole transport layer for an organic electronic device such as a photoelectric conversion element and an organic EL element.

A preferred aspect of the photoelectric conversion element of the present invention is described below.

<Photoelectric Conversion Element>

The photoelectric conversion element of the present invention typically includes a conductive support 1, an electron transport layer 2, a photoelectric conversion layer 3, a hole transport layer 4, and a counter electrode 5, as shown in a schematic cross-sectional view of the FIGURE.

For the purpose of preventing dopant diffusion, a hole transport layer may be inserted between the photoelectric conversion layer 3 and the hole transport layer 4 to form a multi-layer structure that a hole transport layer is inserted.

The photoelectric conversion element of the present invention preferably includes the conductive support 1, the electron transport layer 2, the photoelectric conversion layer 3, the hole transport layer 4, and the counter electrode 5 as shown in the FIGURE, but is not limited thereto. The photoelectric conversion element of the present invention is preferably used as a solar cell, more preferably a perovskite photoelectric conversion element, but is not limited thereto. In the present invention, the perovskite photoelectric conversion element preferably includes the conductive support (electrode) 1, the electron transport layer 2, the photoelectric conversion layer (perovskite layer) 3, the hole transport layer 4, and the counter electrode 5 in this order. The perovskite photoelectric conversion element may include the conductive support, the hole transport layer, the photoelectric conversion layer (perovskite layer), the electron transport layer, and the counter electrode in this order.

<Conductive Support>

In the photoelectric conversion element of the present invention, the conductive support 1 shown in the FIGURE

must have a translucency that allows transmission of light contributing to photoelectric conversion. The conductive support is preferably a conductive substrate because the conductive support is a member having a function of extracting current from the photoelectric conversion layer. Specific examples of a conductive material may include a conductive transparent oxide semiconductor such as tin-doped indium oxide (ITO), zinc-doped indium oxide (IZO), tungsten-doped indium oxide (IWO), zinc aluminum oxide (AZO), fluorine-doped tin oxide (FTO), indium oxide ($In_2O_3$), and indium-tin composite oxide. And, tin-doped indium oxide (ITO), fluorine-doped tin oxide (FTO), and the like are preferably used.

<Electron Transport Layer>

In the photoelectric conversion element of the present invention, the electron transport layer 2 shown in the FIGURE is a layer located between the conductive support 1 and the photoelectric conversion layer (perovskite layer) 3, and the electron transport layer 2 is preferably formed on the conductive support 1. However, it is not limited to specific configurations. The electron transport layer is used to improve an efficiency of electron transfer from the photoelectric conversion layer to the electrode and to block transfer of a hole.

In the present invention, specific examples of a semiconductor forming the electron transport layer may include metal oxide such as tin oxide (SnO, $SnO_2$, $SnO_3$, or the like), titanium oxide ($TiO_2$ or the like), tungsten oxide ($WO_2$, $WO_3$, $W_2O_3$, or the like), zinc oxide (ZnO), niobium oxide ($Nb_2O_5$ or the like), tantalum oxide ($Ta_2O_5$, or the like), yttrium oxide ($Y_2O_3$ or the like), and strontium titanate ($SrTiO_3$ or the like); metal sulfide such as titanium sulfide, zinc sulfide, zirconium sulfide, copper sulfide, tin sulfide, indium sulfide, tungsten sulfide, cadmium sulfide, and silver sulfide; metal selenide such as titanium selenide, zirconium selenide, indium selenide, and tungsten selenide; and an elemental semiconductor such as silicon and germanium, and one or more of these semiconductors are preferably used. In the present invention, one or more selected from tin oxide, titanium oxide, and zinc oxide are preferably used as the semiconductor.

In the present invention, a commercially available paste containing semiconductor fine particles may be used, or a paste prepared by dispersing a commercially available semiconductor fine powder in a solvent (coating solution for electron transport layer) may be used. Specific examples of the solvent used in preparing the paste may include water; an alcoholic solvent such as methanol, ethanol, or isopropyl alcohol; a ketone solvent such as acetone, methyl ethyl ketone, or methyl isobutyl ketone; and a hydrocarbon solvent such as n-hexane, cyclohexane, benzene, or toluene, but are not limited thereto. Also, these solvents can be used singly or as a mixed solvent of two or more.

Examples of a method for dispersing the semiconductor fine powder in the solvent in the present invention include grounding the powder in a mortar or the like and then dispersing the grounded powder, and using a dispersing machine such as a ball mill, a paint conditioner, a vertical bead mill, a horizontal bead mill, or an attritor. When the paste is prepared, a surfactant or the like is preferably added to prevent aggregation of the semiconductor fine particles, and a thickener such as polyethylene glycol is preferably added to increase viscosity.

In the present invention, the electron transport layer can be obtained by using a well-known film-forming method, depending on a material to be formed. As a film-forming method for the electron transport layer, any coating method using a coating solution can be used. Examples include a method in which a film is formed by coating a conductive substrate with a wet coating method such as a spin coating method, an inkjet method, a doctor blade method, a drop casting method, a squeegee method, a screen printing method, a reverse roll coating method, a gravure coating method, a kiss coating method, a roll brush method, a spray coating method, an air knife coating method, a wire barber coating method, a pipe doctor method, an impregnation coating method, and a curtain coating method, and then baking to remove a solvent and additives, a sputtering method, a deposition method, an electrodeposition method, an electrolyte deposition, and a microwave irradiation method, but the present invention is not limited thereto. In the present invention, it is preferable to form a film by a spin coating method using the coating solution for electron transport layer prepared by the above method, but the present invention is not limited thereto. Spin coating conditions can be set as appropriate. An atmosphere in which the film is formed is not limited, and may be in the air.

From a viewpoint of further improving the photoelectric conversion efficiency, a thickness of the electron transport layer is preferably 5 nm to 100 nm, and more preferably 10 nm to 50 nm, when a dense electron transport layer is used. In the present invention, when a porous (mesoporous) metal oxide is used in addition to the dense layer, a thickness thereof is preferably 20 nm to 200 nm, and more preferably 50 nm to 150 nm.

<Photoelectric Conversion Layer>

In the photoelectric conversion element of the present invention, the photoelectric conversion layer (perovskite layer) 3 is preferably formed on the electron transport layer 2 shown in the FIGURE.

In the present invention, when used as a perovskite photoelectric conversion element, the perovskite material that is the photoelectric conversion layer represents a series of materials having a structure represented by a Formula $ABX_3$. Here, A, B, and X each represent an organic cation or a monovalent metal cation, a metal cation, and a halide

anion, and examples include A = $K^+$, $Rb^+$, $Cs^+$, $CH_3NH_3^+$ (hereinafter, MA: methylammonium), NH=$CHNH_2^+$ (hereinafter, FA: formamidinium), $CH_3CH_2NH_3^+$ (hereinafter, EA:ethylammonium); B = Pb, Sn; and X = $I^-$ and Br. More specifically, a layer containing a perovskite material represented by any composition of $MAPbI_3$, $FAPbI_3$, $EAPbI_3$, $CsPbI_3$, $MASnI_3$, $FASnI_3$, $EASnI_3$, $MAPbBr_3$, $FAPbBr_3$, $EAPbBr_3$, $MASnBr_3$, $FASnBr_3$, and $EASnBr_3$, and perovskite materials of a mixed cation and a mixed anion represented by any composition of (FAMA)Pb$(IBr)_3$, K(FAMA)Pb$(IBr)_3$, Rb(FAMA)Pb$(IBr)_3$, and Cs(FAMA)Pb$(IBr)_3$ can be used, but the present invention is not limited thereto. One or more of these perovskite materials are preferably used. A light absorber other than the perovskite material may be contained.

Any coating method can be used as a method for coating the photoelectric conversion layer (perovskite layer) of the photoelectric conversion element of the present invention with a coating solution, and the same method as the film-forming method for the electron transport layer can be used.

A perovskite precursor may be a commercially available material, and in the present invention, is preferably a precursor including lead halide, methylammonium halide, formamidine halide, and cesium halide with any composition, but the perovskite precursor is not limited thereto.

Examples of a solvent for a perovskite precursor solution of the present invention include N,N-dimethylformamide (DMF), dimethylsulfoxide (DMSO), and γ-butyrolactone from a viewpoint of solubility of the precursor, but the solvent is not limited thereto. These solvents may be used singly or in combination of two or more, and a mixed solution of N,N-dimethylformamide and dimethylsulfoxide is preferably used.

In the present invention, an atmosphere during film formation of the photoelectric conversion layer (perovskite layer) is preferably a dry atmosphere, and more preferably a dry inert gas atmosphere in a glove box or the like, because the atmosphare allows for preventing contamination of moisture and producing a highly efficient perovskite solar cell with good reproducibility. And during the film formation, dehydrating with a molecular sieve or the like and using a solvent with a low moisture content are preferable.

In the present invention, from a viewpoint of producing the perovskite material from the precursor, a temperature for heating the photoelectric conversion layer (perovskite layer) with a hot plate or the like is preferably 50°C to 200°C, and more preferably 70°C to 150°C. A heating time is preferably about 10 minutes to 90 minutes, and more preferably about 10 minutes to 60 minutes.

From a viewpoint of further preventing performance deterioration due to defects and peeling, and in order to ensure that the photoelectric conversion layer has a sufficient light absorption rate and an element resistance does not become too high, a thickness of the photoelectric conversion layer (perovskite layer) of the present invention is preferably 50 nm to 1000 nm, and more preferably 300 nm to 700 nm.

<Hole Transport Layer>

In the photoelectric conversion element of the present invention, the hole transport layer 4 shown in the FIGURE is a layer having a function of transporting a hole, and is a layer located between the photoelectric conversion layer (perovskite layer) 3 and the counter electrode 5. The hole transport layer is used to improve an efficiency of hole transfer from the photoelectric conversion layer to the electrode and to block transfer of the electron. The hole transport layer can use, for example, a conductor, a semiconductor, or an organic hole transport material, and may contain an additive for further improving hole transport characteristics. It is desirable to reduce an amount of the additive to be used in the hole transport layer, and the photoelectric conversion element of the present invention also exhibits excellent characteristics without the additive in the hole transport layer.

The hole transport layer of the present invention is a layer containing the compound represented by Formula (1) as the hole transport material. In the hole transport layer of the present invention, the compounds represented by Formula (1) may be used singly or in combination of two or more, and may also be used in combination with another hole transport material that does not belong to the present invention. For preventing dopant diffusion between the photoelectric conversion layer and the hole transport layer, also for forming a multi-layer structure using a layer (hereinafter, also referred to as the intermediate layer) in which the hole transport layer is inserted, the compound represented by Formula (1) can be contained in the intermediate layer, and the hole transport material that does not belong to the present invention can also be used as the hole transport layer.

Specific examples of the another hole transport material that does not belong to the hole transport material of the present invention include a compound semiconductor containing monovalent copper such as CuI, $CuInSe_2$, or CuS; and a compound containing a metal other than copper, such as GaP, NiO, CoO, FeO, $Bi_2O_3$, $MoO_2$, or $Cr_2O_3$, and these metal oxides may be mixed in the hole transport layer or laminated on the hole transport material. Examples of the organic hole transport material include a polythiophene derivative such as poly-3-hexylthiophene (P3HT) or polyethylenedioxythiophene (PEDOT); a fluorene derivative such as 2,2',7,7'-tetrakis-(N,N-di-p-methoxyphenylamine)-9,9'-spirobifluorene (Spiro-OMeTAD); a carbazole derivative such as polyvinylcarbazole; a triphenylamine derivative such as poly[bis(4-phenyl)(2,4,6-trimethylphenyl)amine] (PTAA); a diphenylamine derivative; a polysilane derivative; and a polyaniline derivative.

Any coating method can be used as a method for coating the hole transport layer of the photoelectric conversion element of the present invention with a coating solution, and the same method as the film-forming method for the electron transport layer can be used.

In the present invention, examples of the solvent used in the coating solution for hole transport layer when forming a film include an aromatic organic solvent such as benzene, toluene, xylene, mesitylene, tetralin (1,2,3,4-tetrahydronaphthalene), monochlorobenzene (chlorobenzene), o-dichlorobenzene, m-dichlorobenzene, p-dichlorobenzene, and nitrobenzene; a halogenated alkyl organic solvent such as dichloromethane, chloroform, 1,2-dichloroethane, 1,1,2-trichloroethane, and dichloromethane; a nitrile solvent such as benzonitrile and acetonitrile; an ether solvent such as tetrahydrofuran, dioxane, diisopropyl ether, c-pentyl methyl ether, ethylene glycol dimethyl ether, ethylene glycol diethyl ether, and propylene glycol monomethyl ether; an ester solvent such as ethyl acetate and propylene glycol monomethyl ether acetate; and an alcoholic solvent such as methanol, isopropanol, n-butanol, propylene glycol, 1,3-butanediol, 1,4-butanediol, 2,3-butanediol, cyclohexanol, and 2-n-butoxyethanol, but the present invention is not limited thereto. The above solvents may be used singly or in combination of two or more, and the solvent to be used can be selected depending on the structure. In particular, the aromatic organic solvent and the halogenated alkyl organic solvent are preferably used.

In the present invention, from a viewpoint of further improving the photoelectric conversion efficiency, a film thickness of the hole transport layer is preferably 5 nm to 500 nm, and more preferably 10 nm to 250 nm. In order to prevent dopant diffusion between the photoelectric conversion layer 4 and the hole transport layer 5, even when the hole transport layer has the multi-layer structure in which the hole transport layer is inserted, a total film thickness is preferably the same.

In the present invention, an atmosphere during film formation of the hole transport layer is preferably a dry atmosphere because the atmosphere allows for producing a highly efficient perovskite solar cell with good reproducibility by preventing contamination of moisture. A dehydrated solvent with a moisture content of 10 ppm or less is preferably used.

<Additive>

In the present invention, the hole transport layer may contain a dopant (or oxidizing agent) or a basic compound (or basic additive) as an additive. Adding an additive to the hole transport layer to increase a carrier concentration of the hole transport material in the hole transport layer (doping) leads to an improvement in the conversion efficiency of the photoelectric conversion element. In the present invention, when the hole transport layer contains the dopant and/or the basic additive, which are additives, an amount of the additive is preferably 3.5 equivalents or less with respect to 1 equivalent of the hole transport material. On the other hand, the use of the dopant and/or the basic additive, which are additives, are used, in manufacturing the photoelectric conversion element, may cause processes to be complicated and costs to increase. Also, the use of the dopant and/or the basic additive may lead to low durability and a short life of the element, regarding the characteristics. Therefore, it is desirable to reduce the amount of the additives to be used. The photoelectric conversion element of the present invention exhibits excellent characteristics without containing the additive.

In the present invention, when the dopant is contained, specific examples of the dopant may include lithium bis(trifluoromethylsulfonyl)imide (LiTFSI), bis(trifluoromethanesulfonyl)imide silver, tris(2-(1H-pyrazol-1-yl)-4-tert-butylpyridine)cobalt(III) tri[bis(trifluoromethane)sulfonimide] (FK209), $NOSbF_6$, SbCls, and $SbF_5$. In the present invention, lithium bis(trifluoromethylsulfonyl)imide (LiTFSI) is preferably used, but the present invention is not limited thereto.

In the present invention, when the dopant is used, the dopant is preferably 2.0 equivalents or less, and more preferably 0.5 equivalents or less with respect to 1 equivalent of the hole transport material contained in the hole transport layer.

In the present invention, the basic compound (basic additive) may be contained as the additive for the hole transport layer. In the present invention, when the basic compound is contained, specific examples thereof include 4-tert-butylpyridine (tBP), 2-picoline, and 2,6-lutidine. The basic compound is often used in combination with the dopant. Also in the present invention, when the dopant is used, it is desirable to use the basic compound in combination, and it is preferable to use tert-butylpyridine.

In the present invention, when the basic compound is used, the basic compound is preferably 5 equivalents or less, and more preferably 3 equivalents or less with respect to 1 equivalent of the hole transport material of the present invention.

<Counter Electrode>

In the present invention, the counter electrode 5 shown in the FIGURE is arranged opposite to the conductive support 1 and formed on the hole transport layer 4, so that charges can be exchanged with the hole transport layer. In the photoelectric conversion element of the present invention, it is preferable to provide a metal electrode as the counter

electrode on the hole transport layer 4, and an electron blocking layer made of an organic material or an inorganic compound semiconductor can be added between the hole transport layer 4 and the counter electrode 5.

In the present invention, specific examples of the material used for the counter electrode include a metal such as platinum, titanium, stainless steel, aluminum, gold, silver, nickel, magnesium, chromium, cobalt, or copper, or an alloy thereof. In the above specific examples, it is preferable to use gold, silver, or a silver alloy since they have high electrical conductivity even in a thin film. In order to be less susceptible to sulfidation or chlorination and to improve a stability of a thin film, examples of the silver alloy include a silver-gold alloy, a silver-copper alloy, a silver-palladium alloy, a silver-copper-palladium alloy, and a silver-platinum alloy.

In the present invention, the counter electrode is preferably made of a material that can be formed by a method such as vapor deposition.

When a metal electrode is used as the counter electrode, in order to obtain excellent conductivity, a film thickness thereof is preferably 10 nm or more, and more preferably 50 nm or more.

In the photoelectric conversion element of the present invention, the conductive support serves as a cathode and the counter electrode serves as an anode. Light such as sunlight is preferably applied from a conductive support side. By irradiation of sunlight or the like, the photoelectric conversion layer (perovskite layer) absorbs light and enters an excited state, and generates the electron and the hole. The electron moves through the electron transport layer and the hole moves through the hole transport layer to the electrode, thereby causing current to flow, and functioning as a photoelectric conversion element.

When evaluating performance (characteristics) of the photoelectric conversion element of the present invention, a short-circuit current density, an open-circuit voltage, Fill Factor, and a photoelectric conversion efficiency are measured. The short-circuit current density represents current per 1 $cm^2$ that flows between two terminals when an output terminal is short-circuited, and the open-circuit voltage represents a voltage between the two terminals when the output terminal is open-circuited. The Fill Factor is a value obtained by dividing a maximum output (product of current and voltage) by a product of the short-circuit current density and the open-circuit voltage and is primarily dependent on an internal resistance. The photoelectric conversion efficiency is obtained by dividing a maximum output (W) by a light intensity (W) per 1 $cm^2$ to obtained a value and multiplying the value by 100, which is expressed as a percentage.

The photoelectric conversion element of the present invention can be applied to a perovskite solar cell, various optical sensors, and the like. The photoelectric conversion element including, as the hole transport layer, the hole transport material containing the compound represented by Formula (1) serves as a cell, the required number of cells are arranged to form a module, and predetermined electrical wiring is provided, so that the perovskite solar cell of the present invention is obtained.

Although the preferred embodiment has been described above, the present invention is not limited thereto, and may be modified as appropriate without departing from the scope of the present invention.

## EXAMPLES

Hereinafter, the present invention will be specifically described with reference to Examples, but the present invention is not limited to the following Examples. Compounds obtained in Synthesis Examples were identified by $^1$H-NMR (1H-NMR (nuclear magnetic resonance apparatus manufactured by JEOL Ltd., JNM-ECZ400S/L1 type)).

[Synthesis Example 1]

Synthesis of Compound (A-1)

3,6-Dibromocarbazole (0.65 g, manufactured by TCI), [4-[bis(4-methoxyphenyl)amino]phenyl]boronic acid (1.68 g, manufactured by TCI), 2 M potassium carbonate aqueous solution (10 mL), tetrakistriphenylphosphine palladium (0.06 g, manufactured by Kanto Kagaku Co., Ltd.), and THF (30 mL) were added in a reaction vessel, and the reaction vessel was degassed under reduced pressure. The mixture was heated under reflux for 6 hours, and after completion of the reaction, the reaction solution was added into a beaker containing water (150 mL). After toluene (30 mL) was added to the above solution, liquid separation was performed. An organic layer was dried over magnesium sulfate and then concentrated. A crude product was purified with a silica gel column (toluene:ethyl acetate = 50:1 (volume ratio)) to obtain a compound represented by the following Formula (2) as a white solid (yield: 0.85 g, yield rate: 55%).

$^1$H-NMR (400 MHz, THF-$d_8$):$\delta$ (ppm) = 3.73 (12H), 6.81 (8H), 6.96 (4H), 7.01 (8H), 7.40 (2H), 7.51 (4H), 7.56 (2H), 8.30 (2H), 10.3 (1H).

[Chem. 10]

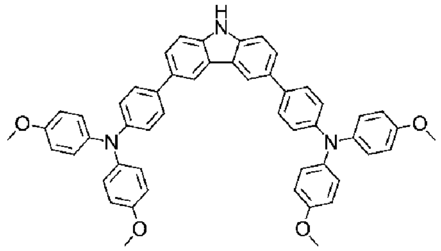

(2)

The compound (0.40 g) of the above Formula (2), sodium hydride (0.03 g, manufactured by Kanto Kagaku Co., Ltd.), and THF (15 mL) were added into a reaction vessel and the mixture was stirred at room temperature for 3 hours. To the mixture was added 2,4-butanesultone (0.08 mL, manufactured by TCI), followed by heating under reflux for 4 hours. The reaction solution was concentrated, followed by dissolving in toluene (60 mL) and then filtering, and the filtrate was concentrated. The crude product was purified with a silica gel column (ethyl acetate) and then recrystallized (acetone:methanol) to obtain a compound represented by the following Formula (A-1) as a pale green solid (yield: 0.40 g, yield rate: 86%).

$^1$H-NMR (400 MHz, DMSO-$d_6$):$\delta$ (ppm) = 1.16 (3H), 1.76 (1H), 2.22 (1H), 2.47 (1H), 3.73 (12H), 4.57 (2H), 6.88 to 6.95 (12H), 7.04 (8H), 7.63 (4H), 7.70 (4H), 8.48 (2H)

[Chem. 11]

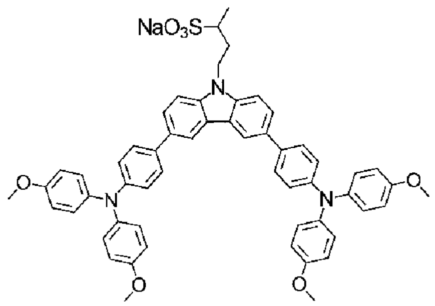

(A−1)

[Synthesis Example 2]

Synthesis of Compound (A-2)

The compound (0.20 g) of the above Formula (2), potassium tert-butoxide (0.06 g, manufactured by Kanto Kagaku Co., Ltd.), and THF (7 mL) were added into a reaction vessel and the mixture was stirred at room temperature for 3 hours. To the mixture was added 2,4-butanesultone (0.04 mL, manufactured by TCI), followed by heating under reflux for 4 hours. The reaction solution was concentrated, followed by dissolving in toluene (30 mL) and then filtering, and the filtrate was concentrated. The crude product was recrystallized (acetone:methanol) to obtain a compound represented by the following Formula (A-2) as a milky white solid (yield: 0.15 g, yield rate: 60%).

$^1$H-NMR (400 MHz, DMSO-$d_6$):$\delta$ (ppm) = 1.16 (3H), 1.76 (1H), 2.22 (1H), 2.40 (1H), 3.75 (12H), 4.58 (2H), 6.89 to 6.95 (12H), 7.05 (8H), 7.63 (4H), 7.70 (4H), 8.47 (2H)

[Chem. 12]

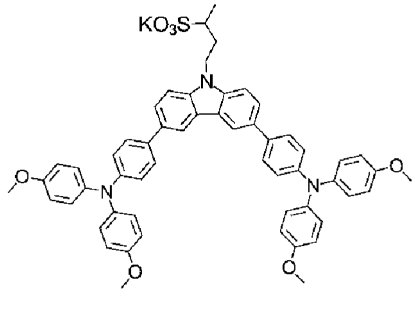

(A−2)

[Synthesis Example 3]

Synthesis of Compound (A-3)

The compound (0.20 g) of the above Formula (2), cesium carbonate (0.19 g, manufactured by Kanto Kagaku Co., Ltd.), and THF (7 mL) were added into a reaction vessel and the mixture was stirred at room temperature for 3 hours. To the mixture was added 2,4-butanesultone (0.04 mL, manufactured by TCI), followed by heating under reflux for 4 hours. The reaction solution was filtered, followed by washing with THF (20 mL) and then concentrating. The concentrated product was dissolved in methanol, followed by filtering, and the filtrate was concentrated. The crude product was recrystallized (acetone:ethanol) to obtain a compound represented by the following Formula (A-3) as a pale green solid (yield: 0.09 g, yield rate: 33%).

$^{1}$H-NMR (400 MHz, DMSO-$d_6$):δ (ppm) = 1.15 (3H), 1.32 (1H), 1.87 (1H), 2.32 (1H), 3.77 (12H), 4.57 (2H), 6.88 to 6.95 (12H), 7.03 (8H), 7.63 (4H), 7.69 (4H), 8.46 (2H)

[Chem. 13]

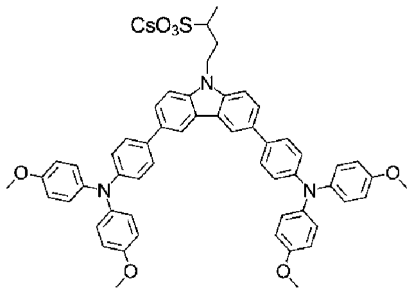

(A−3)

[Synthesis Example 4]

Synthesis of Compound (A-4)

The compound (0.20 g) of the above Formula (2), rubidium carbonate (0.13 g, manufactured by Kanto Kagaku Co., Ltd.), and THF (7 mL) were added into a reaction vessel and the mixture was stirred at room temperature for 3 hours. To the mixture was added 2,4-butanesultone (0.04 mL, manufactured by TCI), followed by heating under reflux for 4 hours. The reaction solution was filtered, followed by washing with THF (20 mL) and then concentrating. The concentrated product was dissolved in methanol, followed by filtering, and the filtrate was concentrated. The crude product was recrystallized (acetone:ethanol) to obtain a compound represented by the following Formula (A-4) as a pale green solid (yield: 0.07 g, yield rate: 27%).

$^{1}$H-NMR (400 MHz, DMSO-$d_6$):δ(ppm) = 1.16 (3H), 1.44 (1H), 2.15 (1H), 2.33 (1H), 3.73 (12H), 4.57 (2H), 6.88 to 6.95 (12H), 7.04 (8H), 7.63 (4H), 7.70 (4H), 8.47 (2H)

[Chem. 14]

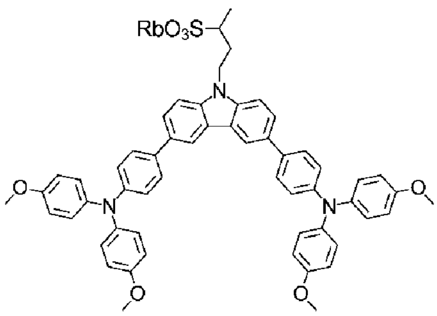

(A−4)

[Synthesis Example 5]

Synthesis of Compound (A-5)

Except that the compound represented by the above Formula (2) was changed to a compound represented by

the following Formula (3), a compound represented by the following Formula (A-5) was obtained as a pale green solid (yield: 0.25 g, yield rate: 81%) in the same manner as in Synthesis Example 1.

$^{1}$H-NMR (400 MHz, DMSO-$d_6$):δ (ppm) = 1.18 (3H), 1.79 (1H), 2.23 (1H), 2.46 (1H), 3.65 (12H), 4.57 (2H), 6.81 (8H), 6.85 (8H), 7.10 (2H), 7.50 (2H), 7.79 (2H)

[Chem. 15]

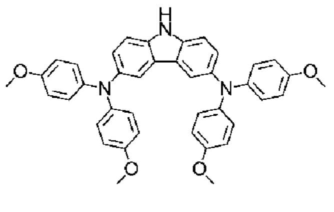
(3)

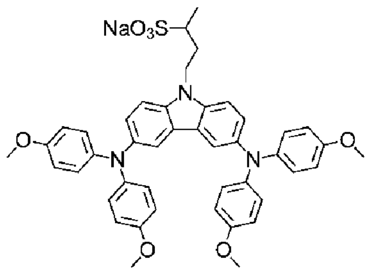
(A−5)

[Synthesis Example 6]

Synthesis of Compound (A-6)

Except that the compound represented by the above Formula (2) was changed to the compound represented by the above Formula (3), a compound represented by the following Formula (A-6) was obtained as a milky white solid (yield: 0.13 g, yield rate: 63%) in the same manner as in Synthesis Example 2.

$^{1}$H-NMR (400 MHz, DMSO-$d_6$):δ(ppm) = 1.18 (3H), 1.79 (1H), 2.23 (1H), 2.40 (1H), 3.63 (12H), 4.57 (2H), 6.81 (8H), 6.85 (8H), 7.10 (2H), 7.50 (2H), 7.78 (2H)

[Chem. 16]

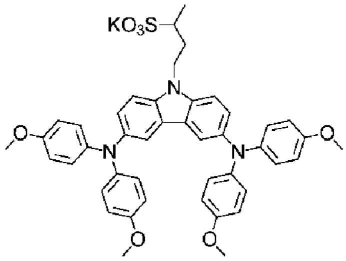
(A−6)

[Synthesis Example 7]

Synthesis of Compound (A-7)

Except that the compound represented by the above Formula (2) was changed to the compound represented by the above Formula (3), a compound represented by the following Formula (A-7) was obtained as a pale green solid (yield: 0.09 g, yield rate: 39%) in the same manner as in Synthesis Example 3.

$^{1}$H-NMR (400 MHz, DMSO-$d_6$):δ(ppm) = 1.17 (3H), 1.35 (1H), 1.88 (1H), 2.32 (1H), 3.65 (12H), 4.57 (2H), 6.81 (8H), 6.85 (8H), 7.09 (2H), 7.49 (2H), 7.77 (2H)

[Chem. 17]

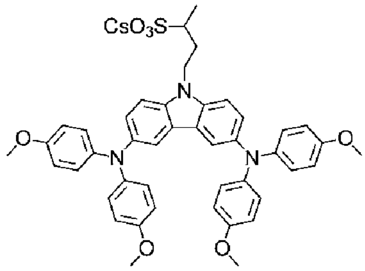

(A-7)

[Synthesis Example 8]

Synthesis of Compound (A-8)

Except that the compound represented by the above Formula (2) was changed to the compound represented by the above Formula (3), a compound represented by the following Formula (A-8) was obtained as a pale green solid (yield: 0.06 g, yield rate: 28%) in the same manner as in Synthesis Example 4.

$^1$H-NMR (400 MHz, DMSO-$d_6$):δ(ppm) = 1.18 (3H), 1.48 (1H), 2.18 (1H), 2.33 (1H), 3.65 (12H), 4.57 (2H), 6.81 (8H), 6.85 (8H), 7.10 (2H), 7.50 (2H), 7.79 (2H)

[Chem. 18]

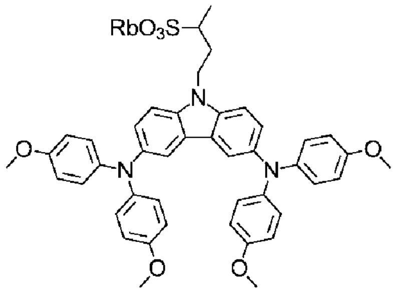

(A-8)

[Synthesis Example 9]

Synthesis of Compound (A-23)

Except that the compound represented by the above Formula (2) was change to a compound represented by the following Formula (4), a compound represented by the following Formula (A-23) was obtained as a pale green solid (yield: 0.38 g, yield rate: 81%) in the same manner as in Synthesis Example 1.

$^1$H-NMR (400 MHz, DMSO-$d_6$):δ(ppm) = 1.16 (3H), 1.76 (1H), 2.22 (1H), 2.47 (1H), 3.73 (12H), 4.57 (2H), 6.88 to 6.95 (12H), 7.00 (8H), 7.49 (2H), 7.60 (4H), 7.85 (2H), 7.96 (2H)

[Chem. 19]

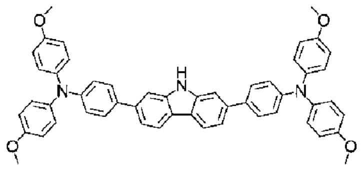

(4)

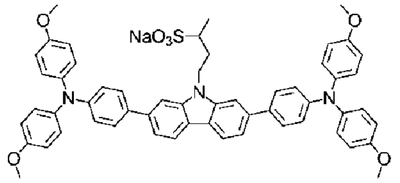

(A-23)

[Synthesis Example 10]

Synthesis of Compound (A-24)

Except that the compound represented by the above Formula (2) was changed to the compound represented by the above Formula (4), a compound represented by the following Formula (A-24) was obtained as a milky white solid (yield: 0.16 g, yield rate: 65%) in the same manner as in Synthesis Example 2.

$^1$H-NMR (400 MHz, DMSO-$d_6$):$\delta$(ppm) = 1.16 (3H), 1.76 (1H), 2.22 (1H), 2.40 (1H), 3.75 (12H), 4.58 (2H), 6.88 to 6.95 (12H), 7.00 (8H), 7.49 (2H), 7.60 (4H), 7.85 (2H), 7.96 (2H)

[Chem. 20]

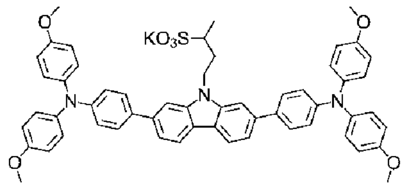

(A-24)

[Synthesis Example 11]

Synthesis of Compound (A-25)

Except that the compound represented by the above Formula (2) was changed to the compound represented by the above Formula (4), a compound represented by the following Formula (A-25) was obtained as a pale green solid (yield: 0.11 g, yield rate: 40%) in the same manner as in Synthesis Example 3.

$^1$H-NMR (400 MHz, DMSO-$d_6$):$\delta$(ppm) = 1.16 (3H), 1.76 (1H), 2.22 (1H), 2.40 (1H), 3.75 (12H), 4.58 (2H), 6.88 to 6.95 (12H), 7.00 (8H), 7.49 (2H), 7.60 (4H), 7.85 (2H), 7.96 (2H)

[Chem. 21]

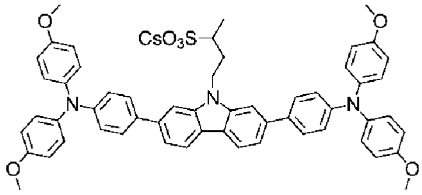

(A-25)

[Synthesis Example 12]

Synthesis of Compound (A-26)

Except that the compound represented by the above Formula (2) was changed to the compound represented

by the above Formula (4), a compound represented by the following Formula (A-26) was obtained as a pale green solid (yield: 0.08 g, yield rate: 31%) in the same manner as in Synthesis Example 4.

$^{1}$H-NMR (400 MHz, DMSO-$d_6$):δ(ppm) = 1.16 (3H), 1.76 (1H), 2.22 (1H), 2.40 (1H), 3.75 (12H), 4.58 (2H), 6.88 to 6.95 (12H), 7.00 (8H), 7.49 (2H), 7.60 (4H), 7.85 (2H), 7.96 (2H)

[Chem. 22]

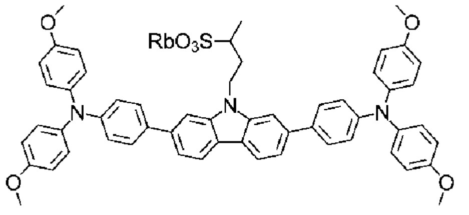

(A−26)

[Synthesis Example 13]

Synthesis of Compound (A-27)

Except that the compound represented by the above Formula (2) was change to a compound represented by the following Formula (5), a compound represented by the following Formula (A-27) was obtained as a pale green solid (yield: 0.28 g, yield rate: 72%) in the same manner as in Synthesis Example 1.

$^{1}$H-NMR (400 MHz, DMSO-$d_6$):δ(ppm) = 1.18 (3H), 1.79 (1H), 2.23 (1H), 2.46 (1H), 3.65 (12H), 4.57 (2H), 6.84 (8H), 6.89 (8H), 6.95 (2H), 7.20 (2H), 7.25 (2H)

[0142] [Chem. 23]

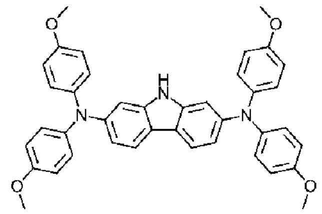

(5)

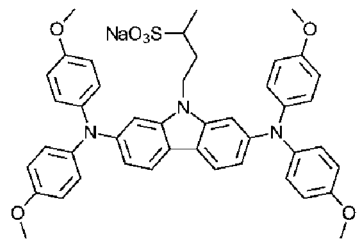

(A−27)

[Synthesis Example 14]

Synthesis of Compound (A-28)

Except that the compound represented by the above Formula (2) was changed to the compound represented by the above Formula (5), a compound represented by the following Formula (A-28) was obtained as a milky white solid (yield: 0.12 g, yield rate: 58%) in the same manner as in Synthesis Example 2.

$^{1}$H-NMR (400 MHz, DMSO-$d_6$):δ(ppm) = 1.18 (3H), 1.79 (1H), 2.23 (1H), 2.40 (1H), 3.63 (12H), 4.57 (2H), 6.84 (8H), 6.89 (8H), 6.95 (2H), 7.20 (2H), 7.25 (2H)

[0145] [Chem. 24]

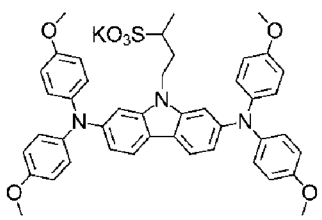

(A−28)

[Synthesis Example 15]

Synthesis of Compound (A-29)

Except that the compound represented by the above Formula (2) was changed to the compound represented by the above Formula (5), a compound represented by the following Formula (A-29) was obtained as a pale green solid (yield: 0.06 g, yield rate: 26%) in the same manner as in Synthesis Example 3.

$^1$H-NMR (400 MHz, DMSO-$d_6$):δ(ppm) = 1.17 (3H), 1.35 (1H), 1.88 (1H), 2.32 (1H), 3.65 (12H), 4.57 (2H), 6.84 (8H), 6.89 (8H), 6.95 (2H), 7.20 (2H), 7.25 (2H)

[0148] [Chem. 25]

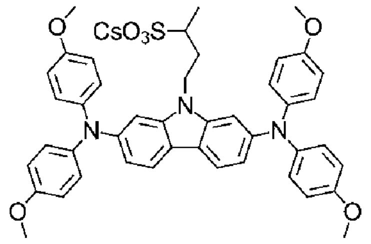

(A−29)

[Synthesis Example 16]

Synthesis of Compound (A-30)

Except that the compound represented by the above Formula (2) was changed to the compound represented by the above Formula (5), a compound represented by the following Formula (A-30) was obtained as a pale green solid (yield: 0.07 g, yield rate: 32%) in the same manner as in Synthesis Example 4.

$^1$H-NMR (400 MHz, DMSO-$d_6$):δ(ppm) = 1.18 (3H), 1.48 (1H), 2.18 (1H), 2.33 (1H), 3.65 (12H), 4.57 (2H), 6.84 (8H), 6.89 (8H), 6.95 (2H), 7.20 (2H), 7.25 (2H)

[Chem. 26]

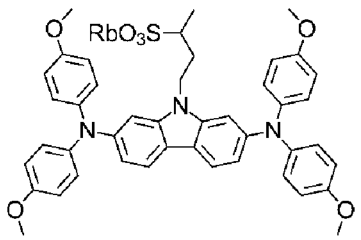

(A−30)

[Synthesis Example 17]

Synthesis of Compound (A-73)

Except that the compound represented by the above Formula (2) was change to a compound represented by the following Formula (6), a compound represented by the following Formula (A-73) was obtained as a pale green solid (yield: 0.29 g, yield rate: 79%) in the same manner as in Synthesis Example 1.

$^1$H-NMR (400 MHz, DMSO-$d_6$):δ(ppm) = 1.18 (3H), 1.84 (1H), 2.28 (1H), 2.53 (1H), 3.70 (24H), 4.67 (2H), 6.83 (16H), 6.89 (16H), 7.12 (4H), 7.40 (4H), 7.73 (6H), 7.75 (2H), 7.94 (2H), 8.10 (6H), 8.79 (2H)

[Chem. 27]

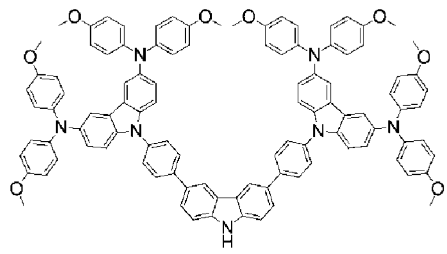

(6)

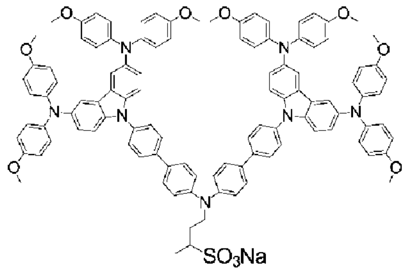

(A−73)

[Synthesis Example 18]

Synthesis of Compound (A-74)

Except that the compound represented by the above Formula (2) was changed to the compound represented by the above Formula (6), a compound represented by the following Formula (A-74) was obtained as a pale green solid (yield: 0.22 g, yield rate: 56%) in the same manner as in Synthesis Example 3.
$^1$H-NMR (400 MHz, DMSO-$d_6$):δ(ppm) = 1.18 (3H), 1.84 (1H), 2.28 (1H), 2.53 (1H), 3.70 (24H), 4.67 (2H), 6.83 (16H), 6.90 (16H), 7.11 (4H), 7.40 (4H), 7.73 (6H), 7.75 (2H), 7.94 2H), 8.10 (6H), 8.79 (2H)

[Chem. 28]

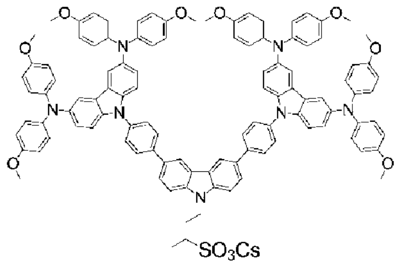

(A−74)

[Synthesis Example 19]

Synthesis of Compound (A-75)

Except that the compound represented by the above Formula (2) was change to a compound represented by the following Formula (7), a compound represented by the following Formula (A-75) was obtained as a pale green solid (yield: 0.21 g, yield rate: 48%) in the same manner as in Synthesis Example 1.
$^1$H-NMR (400 MHz, DMSO-$d_6$):δ(ppm) = 1.18 (3H), 1.89 (1H), 2.31 (1H), 2.56 (1H), 3.70 (24H), 4.81 (2H), 6.84 (16H), 6.90 (16H), 7.12 (4H), 7.42 (4H), 7.62 (2H), 7.74 (8H), 8.17 (6H), 8.31 (2H)

[Chem. 29]

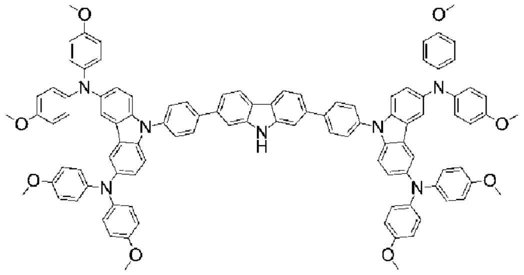

(7)

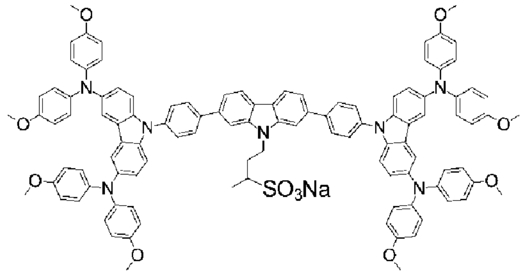

(A−75)

[Synthesis Example 20]

Synthesis of Compound (A-76)

Except that the compound represented by the above Formula (2) was changed to the compound represented by the above Formula (7), a compound represented by the following Formula (A-76) was obtained as a pale green solid (yield: 0.22 g, yield rate: 60%) in the same manner as in Synthesis Example 3.

$^{1}$H-NMR (400 MHz, DMSO-$d_6$):$\delta$(ppm) = 1.19 (3H), 1.89 (1H), 2.30 (1H), 2.53 (1H), 3.73 (24H), 4.81 (2H), 6.82 (16H), 6.90 (16H), 7.11 (4H), 7.41 (4H), 7.63 (2H), 7.73 (8H), 8.15 (6H), 8.28 (2H)

[Chem. 30]

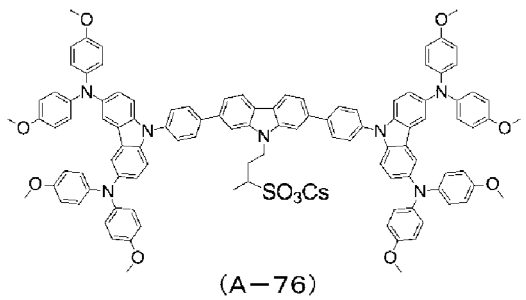

(A−76)

[Comparative Compound 1]

Synthesis of Compound (B-1)

The compound (0.10 g) represented by the above Formula (A-1) was subjected to an ion exchange reaction using an ion exchange resin (manufactured by Sigma-Aldrich) to obtain a compound represented by the following Formula (B-1) as a green solid (yield: 0.07 g, yield rate: 71%).

$^{1}$H-NMR (400 MHz, DMSO-$d_6$):$\delta$(ppm) = 1.16 (3H), 1.76 (1H), 2.22 (1H), 3.73 (12H), 4.57 (2H), 6.88 to 6.95 (12H), 7.04 (8H), 7.63 (4H), 7.70 (4H), 8.48 (2H)

[Chem. 31]

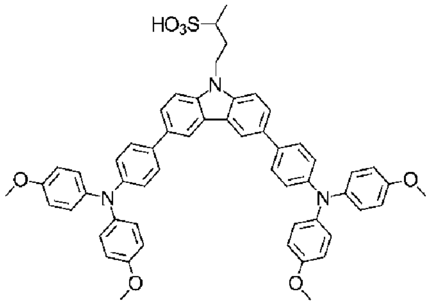

(B−1)

[Example 1]

Preparation of Photoelectric Conversion Element and Evaluation of Photoelectric Conversion Characteristics

An etched glass substrate (conductive support 1, manufactured by Solaronix) coated with a fluorine-doped tin oxide (FTO) thin film was ultrasonically cleaned with isopropyl alcohol and then treated with UV ozone.

A tin oxide dispersion solution containing tin (IV) oxide, 15% in $H_2O$ colloidal dispersion (manufactured by Alfa Aesar) and purified water at a volume ratio of 1:3 was coated on this substrate by spin coating. Thereafter, a tin oxide layer (electron transport layer 2) having a thickness of about 40 nm was formed by heating at 150°C for 30 minutes using a hot plate.

In a glove box under a nitrogen stream, formamidine hydroiodide (1 M, manufactured by Tokyo Chemical Industry Co., Ltd.), lead iodide (II) (1.1 M, manufactured by Tokyo Chemical Industry Co., Ltd.), methylamine hydrobromide (0.2 M, manufactured by Tokyo Chemical Industry Co., Ltd.), and lead bromide (II) (0.2 M, manufactured by Tokyo Chemical Industry Co., Ltd.) were dissolved in a mixed solvent of dimethylformamide and dimethylsulfoxide at a volume ratio of 4:1. A dimethylsulfoxide solution of cesium iodide (1.5 M, manufactured by Tokyo Chemical Industry Co., Ltd.) was added to the above mixture so that a charge weight of cesium was 5% in a composition ratio, and a perovskite precursor solution was prepared.

In the glove box under the nitrogen atmosphere, the prepared perovskite precursor solution was dropped and spin-coated on the tin oxide layer, and 0.3 mL of chlorobenzene was dropped during the spin-coating to form a perovskite precursor film. Thereafter, by heating at 100°C for 1 hour using the hot plate, a $Cs(MAFA)Pb(IBr)_3$ layer (photoelectric conversion layer 3) having a film thickness of about 500 nm was formed.

In a glove box under a nitrogen stream, 50 mM of the compound (A-1), which is the hole transport material obtained in Synthesis Example 1, was dissolved in chlorobenzene to prepare a coating solution for hole transport layer. In the glove box under the nitrogen atmosphere, the $Cs(MAFA)Pb(IBr)_3$ layer (photoelectric conversion layer 3) was spin-coated with the coating solution for hole transport layer to form the hole transport layer 4 having a thickness of about 200 nm.

A gold electrode (counter electrode 5) was provided on the hole transport layer 4 by forming a gold film in a thickness of 80 nm to 100 nm at a degree of vacuum of about $1 \times 10^{-4}$ Pa by a vacuum deposition method, thereby producing a photoelectric conversion element.

Simulated sunlight (AM 1.5, 100 $mW/cm^2$) generated by a white light emission device (OTENTO-SUN SH type, manufactured by Bunkoukeiki Co., Ltd.) was emitted from a conductive support 1 side of the photoelectric conversion element, and current-voltage characteristics were measured by using a source meter (Model 2400 Series Source Meter manufactured by KEITHLEY) to determine the photoelectric conversion efficiency. The determined current-voltage characteristics and the photoelectric conversion efficiency are shown in Table 1.

[Comparative Example 1]

A chlorobenzene solution including 25 mM of bis(trifluoromethanesulfonyl)imide lithium (LiTFSI, 0.5 equivalents) which is a dopant of the additive and 150 mM of 4-tert-butylpyridine which is a basic compound of the additive was prepared as a doping solution. The prepared doping solution and Spiro-OMeTAD (manufactured by Sigma-Aldrich) which is a standard hole transport material represented by the following Formula (B-2) were used to prepare a 50 mM of chlorobenzene solution as the coating solution for hole transport layer. Except that the coating solution for hole transport layer was used, a photoelectric conversion element was produced in the same manner as in Example 1, and the current-voltage characteristics were measured to determine the photoelectric conversion efficiency. The determined current-voltage characteristics and the photoelectric conversion efficiency are shown in Table 1.

[Chem. 32]

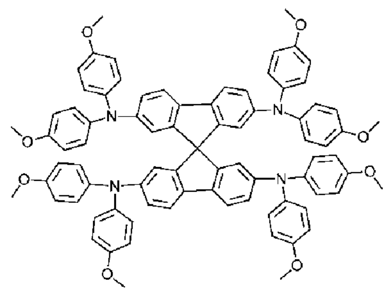

(B−2)

[Comparative Example 2]

Except that the compound represented by the above Formula (B-1) and having a sulfonic acid group without forming a salt is used as the hole transport material, a photoelectric conversion element was produced in the same manner as in Example 1, and the current-voltage characteristics were measured to determine the photoelectric conversion efficiency. The determined current-voltage characteristics and the photoelectric conversion efficiency are shown in Table 1.

[Table 1]

| | Hole transport material | Adding amount (equivalent) of dopant (LiTFSI) | Short-circuit current Jsc [mA/cm$^2$] | Open-circuit voltage Voc [V] | Fill Factor FF | Photoelectric conversion efficiency PCE [%] |
|---|---|---|---|---|---|---|
| Example 1 | A-1 | 0 | 22.75 | 1.04 | 0.71 | 16.69 |
| Comparative Example 1 | B-2 | 0.5 | 22.27 | 1 | 0.69 | 15.38 |
| Comparative Example 2 | B-1 | 0 | 22.71 | 0.76 | 0.4 | 6.88 |

From the results in Table 1, it was found that the use of the hole transport layer containing the compound of the present invention allowed for producing a photoelectric conversion element having more excellent characteristics without the dopant and the basic additive, which are additives, than that using the compound (B-1) containing the dopant and the basic additive, which are additives. Therefore, the photoelectric conversion element containing the compound of the present invention achieves reduction of manufacturing costs by adding no additive and also can be produced by a simple process that does not require the adding. It was found that the use of the hole transport layer containing the compound of the present invention allowed for producing a photoelectric conversion element having more excellent characteristics, than that using the compound (B-2), which forms no salt and is the hole transport material having the sulfonic acid group. Accordingly, it was clarified that the salt-forming hole transport material has better characteristics as a photoelectric conversion element than the non-salt-forming hole transport material.

[Example 2]

Preparation of Photoelectric Conversion Element and Evaluation of Current-Voltage Characteristics

Glass with a FLAT ITO film (conductive support 1, manufactured by Geomatec Co., Ltd.) was subjected to UV ozone treatment after ultrasonic cleaning with isopropyl alcohol.

A tin oxide dispersion solution (electron transport layer coating solution) including tin (IV) oxide, 15% in $H_2O$ colloidal dispersion (manufactured by Alfa Aesar) and purified water at a volume ratio of 1:9 was coated on this ITO film by spin coating. Thereafter, a tin oxide layer (electron transport layer 2) having a thickness of about 20 nm was formed by heating at 150°C for 30 minutes using a hot plate.

In a glove box under a nitrogen stream, formamidine hydroiodide (1 M, manufactured by Tokyo Chemical Industry Co., Ltd.), lead iodide (II) (1.1 M, manufactured by Tokyo Chemical Industry Co., Ltd.), methylamine hydrobromide (0.2 M, manufactured by Tokyo Chemical Industry Co., Ltd.), and lead bromide (II) (0.2 M, manufactured by Tokyo Chemical Industry Co., Ltd.) were dissolved in the mixed solvent of dimethylformamide and dimethylsulfoxide at the volume ratio of 4:1. The dimethylsulfoxide solution of cesium iodide (1.5 M, manufactured by Tokyo Chemical Industry Co., Ltd.) was added to the above mixture so that the charge weight of cesium was 5% in the composition ratio, and the perovskite precursor solution was prepared.

In the glove box under the nitrogen atmosphere, the prepared perovskite precursor solution was dropped and spin-coated on the tin oxide layer, and 0.3 mL of chlorobenzene was dropped during the spin-coating to form the perovskite precursor film. Thereafter, by heating at 100°C for 1 hour using the hot plate, a $Cs(MAFA)Pb(IBr)_3$ layer (photoelectric conversion layer 3) having a film thickness of about 500 nm was formed.

In the glove box under the nitrogen stream, 50 mM of the compound (A-5), which is the hole transport material obtained in Synthesis Example 5, was dissolved in chlorobenzene to prepare a coating solution for hole transport layer.

In the glove box under the nitrogen atmosphere, the $Cs(MAFA)Pb(IBr)_3$ layer (photoelectric conversion layer 3) was spin-coated with the coating solution for hole transport layer to form the hole transport layer 4 having a thickness of about 200 nm.

The gold electrode (counter electrode 5) was formed on the hole transport layer by forming the gold film in a thickness of about 80 nm at a degree of vacuum of about $1 \times 10^{-4}$ Pa by the vacuum deposition method, thereby producing the photoelectric conversion element.

Simulated sunlight (AM 1.5, 100 mW/$cm^2$) generated by the white light emission device (OTENTO-SUN SH type, manufactured by Spectrometer Co., Ltd.) was emitted from the conductive support side of the photoelectric conversion element, and the current-voltage characteristics were measured by using the source meter (Model 2400 Series Source Meter manufactured by KEITHLEY) to determine an initial photoelectric conversion efficiency.

After measuring the current-voltage characteristics, the photoelectric conversion element was stored in a desiccator including silica gel for 28 days, and the current-voltage characteristics were measured again under simulated sunlight irradiation to determine a photoelectric conversion efficiency after 28 days. Table 2 shows the determined photoelectric conversion efficiency (after 28 days) over time.

Table 2 shows a change rate (%) calculated from the following Formula (a-1) using the determined initial photoelectric conversion efficiency and the photoelectric conversion efficiency over time which is the photoelectric conversion efficiency after 28 days.

[Math. 1]

change rate (%) = (photoelectric conversion efficiency over time/initial photoelectric conversion efficiency) * 100 (a-1)

[Example 3]

Except that the compound (A-7) was dissolved in chlorobenzene to be at a concentration of 50 mM instead of the compound (A-5), a photoelectric conversion element was produced in the same manner as in Example 2, and the initial photoelectric conversion efficiency and the photoelectric conversion efficiency after 28 days were determined in the same manner as in Example 2. Table 2 shows the determined photoelectric conversion efficiency (after 28 days) over time. Table 2 shows the change rate (%) calculated from the above Formula (a-1) using the initial photoelectric conversion efficiency and the photoelectric conversion efficiency after 28 days.

[Example 4]

Except that the compound (A-73) was dissolved in chlorobenzene to be at a concentration of 50 mM instead of the compound (A-5), a photoelectric conversion element was produced in the same manner as in Example 2, and the initial photoelectric conversion efficiency and the photoelectric conversion efficiency after 28 days were determined in the same manner as in Example 2. Table 2 shows the determined photoelectric conversion efficiency (after 28 days) over time. Table 2 shows the change rate (%) calculated from the above Formula (a-1) using the initial photoelectric conversion efficiency and the photoelectric conversion efficiency after 28 days.

[Example 5]

Except that the compound (A-74) was dissolved in chlorobenzene to be at a concentration of 50 mM instead of the compound (A-5), a photoelectric conversion element was produced in the same manner as in Example 2, and the initial photoelectric conversion efficiency and the photoelectric conversion efficiency after 28 days were determined in the same manner as in Example 2. Table 2 shows the determined photoelectric conversion efficiency (after 28 days) over time. Table 2 shows the change rate (%) calculated from the above Formula (a-1) using the initial photoelectric conversion efficiency and the photoelectric conversion efficiency after 28 days.

[Example 6]

Except that the compound (A-27) was dissolved in chlorobenzene at 120°C to 30 mM instead of the compound (A-5), a photoelectric conversion element was produced in the same manner as in Example 2, and the initial photoelectric conversion efficiency and the photoelectric conversion efficiency after 28 days were determined in the same manner as in Example 2. Table 2 shows the determined photoelectric conversion efficiency (after 28 days) over time. Table 2 shows the change rate (%) calculated from the above Formula (a-1) using the initial photoelectric conversion efficiency and the photoelectric conversion efficiency after 28 days.

[Example 7]

Except that the compound (A-29) was dissolved in chlorobenzene at 120°C to 30 mM instead of the compound (A-5), a photoelectric conversion element was produced in the same manner as in Example 2, and the initial photoelectric conversion efficiency and the photoelectric conversion efficiency after 28 days were determined in the same manner as in Example 2. Table 2 shows the determined photoelectric conversion efficiency (after 28 days) over time. Table 2 shows the change rate (%) calculated from the above Formula (a-1) using the initial photoelectric conversion efficiency and the photoelectric conversion efficiency after 28 days.

[Comparative Example 3]

In a glove box under a nitrogen stream, 4-tert-butylpyridine and bis(trifluoromethanesulfonyl)imide lithium were dissolved in chlorobenzene as the dopant. Spiro-OMeTAD (manufactured by Sigma-Aldrich), which is a standard hole transport material represented by the above Formula (B-2), was dissolved in the chlorobenzene solution at 50 mM, 3 equivalents of 4-tert-butylpyridine and 0.5 equivalents of the above bis(trifluoromethanesulfonyl)imide lithium were added with respect to (B-2) to prepare a coating solution for hole transport layer. Except for the above, a photoelectric conversion element was produced in the same manner as in Example 2, and the initial photoelectric conversion efficiency and the photoelectric conversion efficiency after 28 days were determined in the same manner as in Example 2. Tables 2 and 3 show the obtained photoelectric conversion efficiency (after 28 days) over time. Tables 2 and 3 show the change rate (%) calculated from the above Formula (a-1) using the initial photoelectric conversion efficiency and the photoelectric conversion efficiency after 28 days.

[Table 2]

| | Hole transport material | Adding amount (equivalent) of dopant (LiTFSI) | Photoelectric conversion efficiency PCE [%] after 28 days | Change rate [%] |
|---|---|---|---|---|
| Example 2 | A-5 | 0 | 14.53 | 104.27 |
| Example 3 | A-7 | 0 | 13.39 | 110.6 |
| Example 4 | A-73 | 0 | 14.93 | 116.24 |
| Example 5 | A-74 | 0 | 8.8 | 75.97 |
| Example 6 | A-27 | 0 | 13.62 | 114.44 |
| Example 7 | A-29 | 0 | 12.54 | 118.15 |
| Comparative Example 3 | B-2 | 0.5 | 10.93 | 64.32 |

From the results in Table 2, it can be seen that even if the dopant is not contained in the hole transport layer, the photoelectric conversion element using the compounds (A-5), (A-7), (A-27), (A-29), (A-73), and (A-74) of the present invention as the hole transport materials exhibits sufficient photoelectric conversion efficiency and less deterioration in element characteristics than the photoelectric conversion element using the compound (B-2) which is the standard hole transport material.

[Example 8]

In a glove box under a nitrogen stream, 4-tert-butylpyridine and bis(trifluoromethanesulfonyl)imide lithium were dissolved in chlorobenzene as the dopant. The compound (A-5), which is the hole transport material obtained in Synthesis Example 5, was dissolved in the chlorobenzene solution at 50 mM, 3 equivalents of 4-tert-butylpyridine and 0.5 equivalents of the above bis(trifluoromethanesulfonyl)imide lithium were added with respect to (A-5) to prepare a coating solution for hole transport layer. Except for the above, a photoelectric conversion element was produced in the same manner as in Example 2, and the initial photoelectric conversion efficiency and the photoelectric conversion efficiency after 28 days were determined in the same manner as in Example 2. Table 3 shows the determined photoelectric conversion efficiency (after 28 days) over time. Table 3 shows the change rate (%) calculated from the above Formula (a-1) using the initial photoelectric conversion efficiency and the photoelectric conversion efficiency after 28 days.

[Example 9]

Except that the compound (A-73) was dissolved in the chlorobenzene solution of the above dopant to be at a concentration of 50 mM instead of the compound (A-5), a photoelectric conversion element was produced in the same manner as in Example 8, and the initial photoelectric conversion efficiency and the photoelectric conversion efficiency after 28 days were determined in the same manner as in Example 8. Table 3 shows the determined photoelectric conversion efficiency (after 28 days) over time after 28 days. Table 3 shows the change rate (%) calculated from the above Formula (a-1) using the initial photoelectric conversion efficiency and the photoelectric conversion efficiency after 28 days.

[Example 10]

Except that the compound (A-74) was dissolved in the chlorobenzene solution of the above dopant to be at a concentration of 50 mM instead of the compound (A-5), a photoelectric conversion element was produced in the same manner as in Example 8, and the initial photoelectric conversion efficiency and the photoelectric conversion efficiency after 28 days were determined in the same manner as in Example 8. Table 3 shows the determined photoelectric conversion efficiency (after 28 days) over time after 28 days. Table 3 shows the change rate (%) calculated from the above Formula (a-1) using the initial photoelectric conversion efficiency and the photoelectric conversion efficiency after 28 days.

[Example 11]

Except that the compound (A-27) was dissolved in the chlorobenzene solution of the above dopant at 60°C to be at a concentration of 50 mM instead of the compound (A-5), a photoelectric conversion element was produced in the same manner as in Example 8, and the initial photoelectric conversion efficiency and the photoelectric conversion efficiency after 28 days were determined in the same manner as in Example 8. Table 3 shows the determined photoelectric conversion efficiency (after 28 days) over time. Table 3 shows the change rate (%) calculated from the above Formula (a-1) using the initial photoelectric conversion efficiency and the photoelectric conversion efficiency after 28 days.

[Example 12]

Except that the compound (A-29) was dissolved in the chlorobenzene solution of the above dopant to be at a concentration of 50 mM instead of the compound (A-5), a photoelectric conversion element was produced in the same manner as in Example 8, and the initial photoelectric conversion efficiency and the photoelectric conversion efficiency after 28 days were determined in the same manner as in Example 8. Table 3 shows the determined photoelectric conversion efficiency (after 28 days) over time. Table 3 shows the change rate (%) calculated from the above Formula (a-1) using the initial photoelectric conversion efficiency and the photoelectric conversion efficiency after 28 days.

[Example 13]

Except that the compound (A-75) was dissolved in the chlorobenzene solution of the above dopant to be at a concentration of 50 mM instead of the compound (A-5), a photoelectric conversion element was produced in the same manner as in Example 8, and the initial photoelectric conversion efficiency and the photoelectric conversion efficiency after 28 days were determined in the same manner as in Example 8. Table 3 shows the determined photoelectric conversion efficiency (after 28 days) over time. Table 3 shows the change rate (%) calculated from the above Formula (a-1) using the initial photoelectric conversion efficiency and the photoelectric conversion efficiency after 28 days.

[Example 14]

Except that the compound (A-76) was dissolved in the chlorobenzene solution of the above dopant to be at a concentration of 50 mM instead of the compound (A-5), a photoelectric conversion element was produced in the same manner as in Example 8, and the initial photoelectric conversion efficiency and the photoelectric conversion efficiency after 28 days were determined in the same manner as in Example 8. Table 3 shows the determined photoelectric conversion efficiency (after 28 days) over time. Table 3 shows the change rate (%) calculated from the above Formula (a-1) using the initial photoelectric conversion efficiency and the photoelectric conversion efficiency after 28 days.

[Table 3]

| | Hole transport material | Adding amount of dopant (LiTFSI) | Photoelectric conversion efficiency PCE after 28 days | Change rate [%] |
|---|---|---|---|---|
| Example 8 | A-5 | 0.5 | 11.72 | 151.71 |
| Example 9 | A-73 | 0.5 | 12.05 | 160.77 |
| Example 10 | A-74 | 0.5 | 10.76 | 178.85 |
| Example 11 | A-27 | 0.5 | 11.01 | 113.7 |
| Example 12 | A-29 | 0.5 | 10.32 | 158.21 |
| Example 13 | A-75 | 0.5 | 6.11 | 260.12 |
| Example 14 | A-76 | 0.5 | 6.11 | 153.03 |
| Comparative Example 3 | B-2 | 0.5 | 10.93 | 64.32 |

From the results in Table 3, it can be seen that the photoelectric conversion element using the compounds (A-5), (A-27), (A-29), (A-73), (A-74), (A-75), and (A-76) of the present invention as the hole transport materials exhibits sufficient photoelectric conversion efficiency than the photoelectric conversion element using the compound (B-2) which is the standard hole transport material, and even if the hole transport layer contains a dopant, the characteristics of the photoelectric conversion element of the present invention do not deteriorate.

Although the present invention has been described in detail with reference to specific embodiments, it is apparent to those skilled in the art that various changes and modifications can be made without departing from the spirit and scope of the present invention.

The present application is based on Japanese Patent Application No. 2021-057864 filed on March 30, 2021, the entire contents of which are incorporated herein by reference. All the references cited herein are incorporated as a whole.

INDUSTRIAL APPLICABILITY

A compound having a sulfonate group according to the present invention is useful for a photoelectric conversion element having good photoelectric conversion efficiency, and allows for providing clean energy as a solar cell that can efficiently convert solar energy into electrical energy, and can also be applied to an organic EL, an image sensor, and the like, when used as a hole transport layer.

REFERENCE SIGNS LIST

1 conductive support
2 electron transport layer
3 photoelectric conversion layer
4 hole transport layer
5 counter electrode

## Claims

1. A compound represented by the following Formula (1),

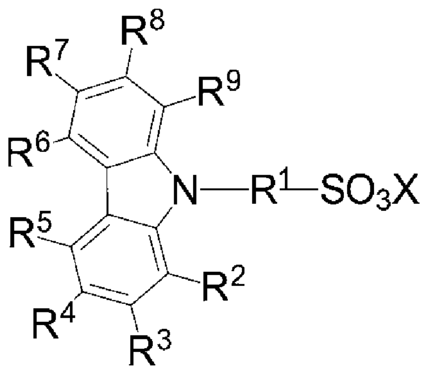

(1)

where, $R^1$ represents:

a linear or branched alkyl group having 1 to 18 carbon atoms that optionally has a substituent group;
a linear or branched alkenyl group having 2 to 20 carbon atoms that optionally has a substituent group;
a linear or branched alkynyl group having 2 to 20 carbon atoms that optionally has a substituent group;
a cycloalkyl group having 3 to 12 carbon atoms that optionally has a substituent group;
an aromatic hydrocarbon group having 6 to 36 carbon atoms that optionally has a substituent group; or
a heterocyclic group having 5 to 36 ring atoms that optionally has a substituent group,
X represents a monovalent cation excluding a hydrogen ion, and
$R^2$ to $R^9$ each independently represent:

a hydrogen atom;
a linear or branched alkyl group having 1 to 18 carbon atoms that optionally has a substituent group;
a linear or branched alkenyl group having 2 to 20 carbon atoms that optionally has a substituent group;
a linear or branched alkynyl group having 2 to 20 carbon atoms that optionally has a substituent group;
a cycloalkyl group having 3 to 12 carbon atoms that optionally has a substituent group;
a linear or branched alkoxy group having 1 to 20 carbon atoms that optionally has a substituent group;
a cycloalkoxy group having 3 to 10 carbon atoms that optionally has a substituent group;
a linear or branched alkoxycarbonyl group having 1 to 18 carbon atoms that optionally has a substituent group;
a thio group having 1 to 18 carbon atoms that optionally has a substituent group;
an amino group having 1 to 20 carbons that optionally has a substituent group;
an aromatic hydrocarbon group having 6 to 36 carbon atoms that optionally has a substituent group; or
a heterocyclic group having 5 to 36 ring atoms that optionally has a substituent group.

**2.** The compound according to claim 1, wherein, in Formula (1), $R^1$ represents the linear or branched alkyl group having 1 to 18 carbon atoms that optionally has a substituent group.

**3.** The compound according to claim 1 or 2, wherein, in Formula (1), at least one of $R^2$ to $R^9$ represents the aromatic hydrocarbon group having 6 to 36 carbon atoms that optionally has a substituent group or the amino group having 1 to 20 carbons that optionally has a substituent group.

**4.** The compound according to any one of claims 1 to 3, wherein, in Formula (1), X represents an alkali metal ion or an ammonium ion that optionally has a substituent group.

**5.** The compound according to claim 4, wherein, in Formula (1), the alkali metal ion is at least one selected from the group consisting of a sodium ion, a potassium ion, a rubidium ion, and a cesium ion.

**6.** A hole transport material comprising the compound according to any one of claims 1 to 5.

**7.** A hole transport material composition for a photoelectric conversion element comprising the hole transport material according to claim 6.

**8.** A photoelectric conversion element using the hole transport material composition for photoelectric conversion according to claim 7.

FIGURE

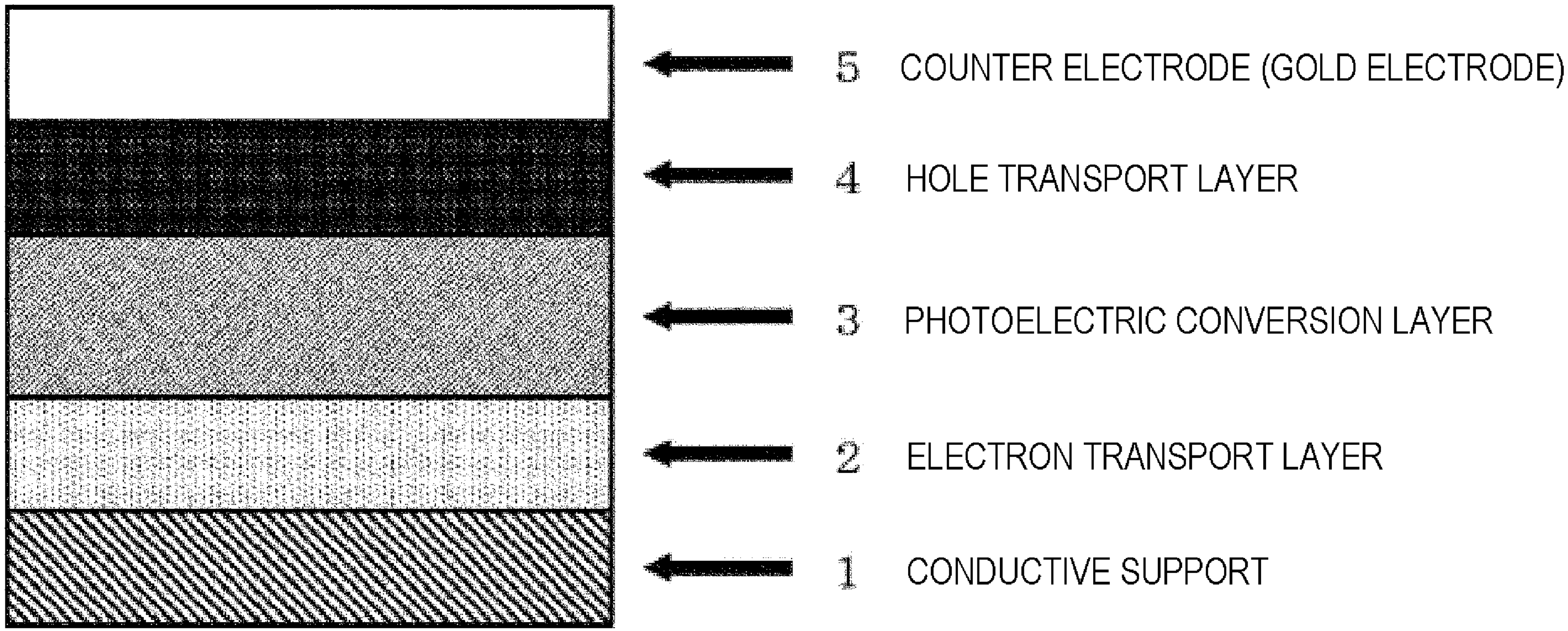
5 COUNTER ELECTRODE (GOLD ELECTRODE)
4 HOLE TRANSPORT LAYER
3 PHOTOELECTRIC CONVERSION LAYER
2 ELECTRON TRANSPORT LAYER
1 CONDUCTIVE SUPPORT

## INTERNATIONAL SEARCH REPORT

International application No.
**PCT/JP2022/014732**

**A. CLASSIFICATION OF SUBJECT MATTER**

***C07D 209/86***(2006.01)i; ***H01L 51/46***(2006.01)i
FI: C07D209/86 CSP; H01L31/04 168; H01L31/04 154C; H01L31/04 154B

According to International Patent Classification (IPC) or to both national classification and IPC

**B. FIELDS SEARCHED**

Minimum documentation searched (classification system followed by classification symbols)

C07D209/86; H01L51/46

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | LAV, T. X. et al., P and n dopable semi-interpenetrating polymer networks, Journal of Solid State Electrochemistry, 2007, vol. 11, no. 6, pp. 859-866, DOI 10.1007/s10008-006-0233-9<br>abstract, p. 864, 865, fig. 9 | 1-8 |
| X | HIDAKA, H. et al., Fluorescence probe studies on alkylcarbazolesulfonates in aqueous solution, Journal of the Chemical Society, Chemical Communications, 1983, no. 3, pp. 99-101, DOI 10.1039/c39830000099<br>p. 99, fig. 1-3 | 1-8 |
| X | ERDOGAN, A. et al., Surface modification of CdSeS nanocrystals for polymer hybrid solar cells, RSC Advances, 2016, vol. 6, no. 33, pp. 27627-27631, DOI 10.1039/C5RA27735C<br>abstract, introduction, fig. 1-5, table 1 | 1-8 |
| X | JP 9-12915 A (DOJINDO LABORATORIES) 14 January 1997 (1997-01-14)<br>table 1 | 1-5 |
| X | WO 2017/201482 A1 (NITTO DENKO CORP.) 23 November 2017 (2017-11-23)<br>example 1.1.6 | 1-5 |

☑ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 May 2022** | **14 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2022/014732

C. DOCUMENTS CONSIDERED TO BE RELEVANT

| Category* | Citation of document, with indication, where appropriate, of the relevant passages | Relevant to claim No. |
|---|---|---|
| X | WO 2020/008957 A1 (FUJIFILM CORP.) 09 January 2020 (2020-01-09)<br>paragraphs [0229]-[0233] | 1-5 |
| A | CN 105384917 A (SOUTH CHINA UNIVERSITY OF TECHNOLOGY) 09 March 2016 (2016-03-09)<br>entire text | 1-8 |
| A | SHANG, R. et al., Disodium Benzodipyrrole Sulfonate as Neutral Hole-Transporting Materials for Perovskite Solar Cells, Journal of the American Chemical Society, 2018, vol. 140, no. 15, pp. 5018-5022, DOI 10.1021/jacs.8b01783<br>entire text | 1-8 |
| A | MAGOMEDOV, A. et al., Self-Assembled Hole Transporting Monolayer for Highly Efficient Perovskite Solar Cells, Advanced Energy Materials, 2018, vol. 8, no. 32, p. 1801892, DOI 10.1002/aenm.201801892<br>entire text | 1-8 |
| A | WO 2019/207029 A1 (HELMHOLTZ-ZENTRUM BERLIN FUR MATERIALIEN UND ENERGIE GMBH) 31 October 2019 (2019-10-31)<br>entire text | 1-8 |

Form PCT/ISA/210 (second sheet) (January 2015)

**INTERNATIONAL SEARCH REPORT**
**Information on patent family members**

International application No.
**PCT/JP2022/014732**

| Patent document cited in search report | Publication date (day/month/year) | Patent family member(s) | Publication date (day/month/year) |
|---|---|---|---|
| JP 9-12915 A | 14 January 1997 | (Family: none) | |
| WO 2017/201482 A1 | 23 November 2017 | JP 2019-516549 A<br>US 2020/0261861 A1<br>EP 3458183 A1<br>KR 10-2019-0010624 A<br>CN 109890488 A | |
| WO 2020/008957 A1 | 09 January 2020 | KR 10-2021-0005669 A | |
| CN 105384917 A | 09 March 2016 | (Family: none) | |
| WO 2019/207029 A1 | 31 October 2019 | US 2021/0234101 A1<br>EP 3784678 A1<br>CN 112469727 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017104792 A
- JP 2018135255 A
- JP 2021057864 A


### Non-patent literature cited in the description


- *J. Am. Chem. Soc.,* 2009, vol. 131, 6050-6051
- *Science,* 2012, vol. 388, 643-647
- *Chem. Sci,* 2019, vol. 10, 6748-6769
- *Adv. Funct. Mater,* 2019, vol. 29 (24), 1901296
- *J. Am. Chem. Soc.,* 2018, vol. 140 (48), 16720-16730